# EUROPEAN PATENT APPLICATION

(11) **EP 4 799 560 A1**
(43) Date of publication of application: **02.09.2026**
(21) Application number: 25161133.1
(22) Date of filing: 28.02.2025
(51) Int. Cl.: A61B 5/024, A61B 5/0265, A61B 5/0507, A61B 5/08, A61B 5/11, A61B 5/113, A61B 5/00, A61B 5/05, A61B 5/0205

(54) **SYSTEM COMPRISING TWO RADIOFREQUENCY ANTENNAS IN AN OPPOSING CONFIGURATION FOR DETERMINING A PHYSIOLOGICAL PARAMETER OF A SUBJECT**

(71) Applicant: UMC Utrecht Holding B.V., 3584 CS Utrecht (NL)
(72) Inventor: Koloskov, Vladislav Borisovich, 3704 HE Zeist (NL); van den Berg, Cornelis Antonius Theodorus, 3514 XH Utrecht (NL); Steensma, Bart Romke, 3523 BD Utrecht (NL)
(74) Representative: Eisenführ Speiser

(57) **Abstract**

The invention relates to a system 1 for determining a physiological parameter like a stroke volume of the heart of a subject 7. A measurement device 8 includes a) an RF antenna module 3 and b) an RF instrument 2 configured to transmit RF power into the RF antenna module 3, to receive an RF signal from the RF antenna module 3 and to provide a motion signal that is related to a mechanical movement of a structure 6 within the subject based on the received RF signal. A first RF antenna 4 and a second RF antenna 5 of the RF antenna module are used in an opposing configuration such that electromagnetic phase profiles of the RF antennas 4, 5 at least partly cancel out, wherein the subject is located in between the opposing RF antennas 4, 5. The physiological parameter is determined based on the provided motion signal.

## Description

### FIELD OF THE INVENTION

The invention relates to a system, method and computer program for determining a physiological parameter of a subject. The invention relates further to a measurement device and a determination device for determining a physiological parameter of a subject, wherein the measurement device is configured to provide a motion signal and the determination device is configured to determine the physiological parameter based on the provided motion signal. Moreover, the invention relates to a radiofrequency (RF) antenna of the measurement device, a use of the RF antenna, a set of a first RF antenna and a second RF antenna, and a method and computer program for controlling the measurement device. Furthermore, the invention relates to a computer program for controlling the determination device. The invention also relates to a training system, training method and training computer program for training a model to be used by the determination device for determining the physiological parameter of the subject. The physiological parameter preferentially is a heart-related physiological parameter or a lung-related physiological parameter.

### BACKGROUND OF THE INVENTION

Diagnostic RF sensing devices, in particular wearable RF sensing devices, are known, which determine physiological parameters such as the respiratory rate, the respiratory volume or the heart rate. However, the accuracy of determining the physiological parameters might be diminished due to, for instance, a non-optimal placement of an RF sensing device on a subject's body, especially a non-optimal placement of a RF sensing device relative to a structure to be monitored like the heart.

### SUMMARY OF THE INVENTION

It is an object of the present invention to provide a system, method and computer program which allow for an improved determination of a physiological parameter of a subject. It is a further object of the present invention to provide a measurement device and a determination device for determining a physiological parameter of the subject, wherein the determination device is configured to allow for an improved determination of the physiological parameter based on a motion signal provided by the measurement device. The invention also relates to an RF antenna of the measurement device, a use of the RF antenna, a set of a first RF antenna and a second RF antenna, and a method and computer program for controlling the measurement device which allow for the improved determination of the physiological parameter. Furthermore, the invention relates to a computer program for controlling the determination device. Moreover, the invention relates to a training system, method and computer program for training a model to be used by the system and the determination device for allowing for the improved determination of the physiological parameter. The invention also relates to a further system for determining a physiological parameter of a subject

In a first aspect of the present invention a system for determining a physiological parameter of a subject is presented, the system comprising:
- a measurement device including a) an RF antenna module comprising a first RF antenna and a second RF antenna and b) an RF instrument connected to the RF antenna module and configured to transmit RF power into the RF antenna module, to receive an RF signal from the RF antenna module and to provide a motion signal that is related to a mechanical movement of a structure within the subject based on the received RF signal,
- a determination device configured to determine the physiological parameter based on the provided motion signal, wherein the determination device comprises a model providing module configured to provide a model that provides, as an output, a physiological parameter if, as an input, a motion signal is provided, and a processor configured to determine the physiological parameter based on the provided model and the provided motion signal,
wherein the RF antenna module is configured to use the RF antennas in an opposing configuration such that electromagnetic phase profiles of the RF antennas at least partly cancel out each other, wherein in use the subject is located in between the opposing RF antennas.

Since the RF antenna module is configured to use the at least two RF antennas in an opposing configuration such that electromagnetic phase profiles of the at least two RF antennas at least partly cancel out each other, wherein in use the subject is located in between the opposing RF antennas, the spatial sensitivity of determining the physiological parameter can be more uniform over the structure. This allows for an improved determination of the physiological parameter. In particular, this can reduce the dependence of the determined physiological parameter on the placement of the at least two RF antennas relative to the structure of the subject like the heart.

The RF antennas of the system for determining a physiological parameter are preferentially configured such that electromagnetic phase profiles of the RF antennas at least partly cancel out each other, if they are used in an opposing configuration, wherein in use the subject is located in between the opposing RF antennas.

The electromagnetic phase profile preferentially refers to a spatial phase distribution of the complex electric field that could be emitted by the RF antenna. The time dependent scattered signal *S_{ij,t}* from transmitting RF antenna *j* to receiving RF antenna *i*, which is modulated by physiological motion, is preferably defined by the following formula as described in the article "Frequency-domain integral equations of scattering for complex scalar responses." by Beaverstone, A. S., IEEE Transactions on Microwave Theory and Techniques, Volume 65, Issue 4, pages 1120-1132 (2017): ${S}_{ij , t} - {S}_{ij , 0} = Δ {S}_{ij , t} = \frac{jω}{2 {a}_{i} {a}_{j}^{∗}} {\int }_{V} \left({ϵ}_{t , r}-{ϵ}_{0 , r}\right) {E}_{0 , r , i} ⋅ {E}_{t , r , j} dV$

In equation 1, Δ*S_{ij,t}* is the change in the scattering coefficient Sᵢⱼ at timepoint t compared to reference timepoint 0, wherein this change is caused by physiological motion. In addition, this change can also be caused by a change in tissue dielectric properties. Note that by setting *i = j*, a reflection coefficient *S*₁₁ is evaluated, while by setting *i* ≠ *j*, a coupling coefficient is evaluated. The normalization term in equation 1 includes the operating frequency *ω* and the forward power *aᵢ, aⱼ* transmitted from an excitation source toward the respective antenna. The terms *ε* and *E* represent the spatiotemporal distributions of the complex tissue permittivity and electric field transmitted by the respective antennas. When *E_{t,r,j}* and *E*_{0*,r,i*} have spatial phase distributions that cancel out, and the highly intense peaks of the respective electric field magnitude are at different locations, the product *E*_{0,*r,i*} · *E_{t,r,j}* is a standing wave with uniform electric field magnitude and phase. It shall be noted that equation 1 allows for defining the scattering coefficients *S_{ij,t}* from a transmitting RF antenna *j* to a receiving RF antenna *i* in terms of the complex electric fields that could be emitted by the RF antennas. However, for an experimental measurement of the scattering coefficients it is not necessary that both RF antennas actually emit an electric field. For instance, it can be sufficient if only the transmitting RF antenna actually emits an electric field in the experiment.

Preferentially, the RF antenna module is configured to use the at least two RF antennas in an opposing configuration such that electromagnetic phase profiles of the at least two RF antennas almost completely cancel out each other. A maximum phase variation within a region of interest is preferably smaller than *π*/2, more preferably smaller than *π*/4. The region of interest can be, for instance, the whole thorax, an organ like the heart or the lungs, or a substructure of an organ such as the ventricle, atrium or arteries and veins.

The opposing configuration is a configuration in which the RF antennas are arranged on opposite sides of the subject, without or with a distance to the subject's body. Preferentially, the RF antennas are placed on opposite sides, for instance, the front side and the back side, of the thorax of the subject. In an embodiment, the measurement device is configured such that, when used, a virtual line from a center point of the first RF antenna to a center point of the second RF antenna intersects the structure within the subject. In an embodiment, the measurement device is configured such that, when used, the center point of the first RF antenna is positioned on the front side of the thorax above the heart and the center point of the second RF antenna is positioned on the back side of the thorax. For instance, the center point of the first RF antenna can be positioned left to the sternum, according to precordial electrocardiography lead placement V2 as defined further below. With this placement, the first RF antenna is positioned directly above the heart, thereby allowing to determine a heart-related physiological parameter even more accurately like the heart rate or the stroke volume. The center point of the second RF antenna can be positioned on the back side of the thorax above the heart as well, so that a virtual line from the center point of the first RF antenna to the center point of the second RF antenna intersects the heart. When seen in the coronal or frontal plane, the center point of the second RF antenna is preferably placed within a 10 cm diameter circle around the center point of the first RF antenna, more preferably within a 2 cm diameter circle around the center point of the first RF antenna. With this placement, the heart is positioned in between the first RF antenna and the second RF antenna, thereby allowing to further increase the accuracy of determining a heart-related physiological parameter.

In order to further increase the accuracy of determining a heart-related physiological parameter related to various substructures of the heart, the first RF antenna can be placed on the front side of the thorax according to precordial lead positions in electrocardiography. The precordial lead positions in electrocardiography can be defined as follows:
- V1 - Fourth intercostal space (ICS), right of the sternum
- V2 - Fourth ICS, left of the sternum
- V3 - Midway between V2 and V4
- V4 - Fifth ICS, midclavicular line (just below the left nipple in men)
- V5 - Fifth ICS, anterior axillary line (in line with V4 but further left)
- V6 - Fifth ICS, midaxillary line (in line with V4 and V5, further left).

For increasing sensitivity to the right ventricle, the first RF antenna can be placed at precordial lead position V1. For increasing sensitivity to the whole heart, the antenna can be placed at precordial lead position V2. For increasing sensitivity to the left ventricle, the antenna can be placed on any of precordial lead positions V4, V5 and V6.

The RF antenna module can comprise more than the first RF antenna und the second RF antenna. For instance, the RF antenna module can comprise more than two RF antennas, wherein at least one first RF antenna is configured to be used on a side of the subject and at least one second RF antenna is configured to be used on an opposing side of the subject, and wherein the RF antennas are configured such that the electromagnetic phase profiles of the RF antennas at least partly cancel out each other.

Moreover, since the RF instrument and the RF antenna module are configured to provide a motion signal that is related to a mechanical movement of a structure like an organ within the subject, i.e., since the RF instrument and the RF antenna module are configured such that the measurement region, which is the region in which the measurement device can sense mechanical motion, covers the structure, the mechanical movement of the structure within the subject directly influences the provided motion signal. Moreover, since this directly influenced motion signal is used by the determination device for determining the physiological parameter, the physiological parameter can be determined with an increased sensitivity.

This is in contrast to the measurement described in, for instance, the articles "Wearable radio-frequency sensing of respiratory rate, respiratory volume, and heart rate" by P. Sharma et al., npj Digital Medicine 3, volume 98, pages 1 to 10 (2020) and "Microwave apexcardiography" by J. Lin et al., IEEE T-MTT 6, volume 27, pages 618 to 620 (1979), in which the measured signal is related to electrical changes close to the skin surface only, i.e., it is not related to mechanical motion of a structure within the subject. For instance, if the structure is the heart, the RF radiation does not penetrate into the heart due to the high RF transmit frequency, which limits the sensitivity. Furthermore, the measurements described in these articles do not allow to determine many different cardiac parameters. For instance, it is not possible to quantify stroke volume of the heart with the measurements disclosed in these articles.

The RF instrument can be configured to directly provide the received RF signal as the motion signal. For example, the RF instrument can be configured to receive an RF signal from the antenna module and to provide directly the received RF signal to the determination device as the motion signal. However, the RF instrument can also be configured to process the received RF signal and to provide the processed RF signal as the motion signal. Moreover, the RF instrument preferentially is configured to separate the received RF signal from the transmitted RF signal, i.e. from the RF power transmitted into the RF antenna module, if required. In a preferred embodiment the RF instrument is a vector network analyzer. Moreover, in an embodiment the RF instrument is configured to use a bi-directional coupler or to carry out the transmitting and receiving procedure after each other, in order to separate the transmitted RF signal from the received RF signal.

Preferentially, the measurement device is configured to be worn by the subject. However, it is also possible that the measurement device is not configured to be worn by the subject. The measurement device can also be configured to be arranged on a wall or to be arranged on a rack, stage or the like, wherein the subject can be arranged in between the opposing RF antennas of the measurement device for determining the physiological parameter.

The processor can be configured to determine one or more heart-related physiological parameters. For instance, the processor can be configured to determine one or more physiological parameters indicative of intracardiac volume, in particular one or more of stroke volume, end-diastolic volume, end-systolic volume and ejection fraction. Furthermore, the processor can be configured to determine one or more physiological parameters indicative of intracardiac pressure, in particular left ventricular pressure and/or right ventricular pressure. The physiological parameters indicative of intracardiac pressure can refer to pressures reached at a specified phase of the cardiac cycle, for instance, at the end of the diastole or at the end of the systole. Additionally or alternatively, the processor can be configured to determine the heart rate. Preferentially, the processor is configured to determine at least one of the heart rate and the stroke volume.

The processor can be configured to determine one or more lung-related physiological parameters. For instance, the processor can be configured to determine one or more physiological parameters indicative of pulmonary volume, in particular one or more of total lung capacity, tidal volume, functional residual capacity and residual volume. Furthermore, the processor can be configured to determine one or more physiological parameters indicative of pulmonary pressure. Moreover, the processor can be configured to determine one or more physiological parameters indicative of pulmonary ventilation. Additionally or alternatively, the processor can be configured to determine the respiratory rate. Preferentially, the processor is configured to determine at least one of the respiratory rate and the tidal volume.

Preferentially, the processor is configured to determine at least one heart-related physiological parameter and at least one lung-related physiological parameter. For instance, the processor can be configured to determine at least one of the heart rate and the stroke volume as the heart-related physiological parameter and at least one of the respiratory rate and the tidal volume as the lung-related physiological parameter.

The measurement device preferentially comprises a transmitter configured to transmit the motion signal to the determination device. In particular, the measurement device and the determination device can be separate devices which are connected via a wireless data connection like Bluetooth. However, the measurement device and the determination device can also be separate devices which are connected via a wired data connection. The measurement device and the determination device can also be integrated, at least partially, in the same device and, for example, share functional units such as processors, storages, power supplies, et cetera. For instance, the RF instrument of the measurement device can comprise RF hardware which is dedicated to generating, receiving and/or analyzing RF signals. Moreover, the RF instrument can be configured to carry out the steps of the determination device for determining the physiological parameter. For instance, it can comprise a processor configured to carry out the steps of the determination device, wherein this processor could then also be regarded as being a processor of the determination device. In this way, in an example, the RF instrument and the determination device can be integrated.

In a preferred embodiment, the model providing module is a storage like a storage of a mobile device or of a personal computer, in which the model is stored and from which the model can be obtained, and the processor can be a processor of the mobile device or of the personal computer, respectively. The mobile device can be, for instance, a smartphone, a tablet computer or a laptop.

The model providing module can be a storage, as mentioned above, in which the model is stored and from which the model can be obtained, but the model providing module can also be a receiving unit configured to receive the model from another device like another storage. It is also possible that the model providing module is configured to generate the model or adapt a present model by training or calibration and to provide the created or adapted model to the processor.

The RF antenna module is matched to the characteristic impedance of the RF instrument which generally is 50 Ω. Moreover, preferentially the reflection coefficient of the RF antenna module is lower than -3 dB and further preferred lower than -10 dB for the operating frequency. In particular, the reflection coefficient of the first RF antenna and/or the second RF antenna is preferentially lower than -3 dB and further preferred lower than -10 dB for the operating frequency. Furthermore, if the measurement device should be used with several operation frequencies, it is desirable that the RF antenna module has a large bandwidth, which can be defined as the frequency span within which the reflection coefficient is lower than -3 dB. In an embodiment, the RF antenna module is constructed such that it has multiple resonance frequencies within a range spanned by the bandwidth. Corresponding RF antenna modules having multiple resonance frequencies are described further below.

Moreover, in a preferred embodiment the RF instrument and the first RF antenna and the second RF antenna are configured such that an inter-element coupling between the two RF antennas is below a predefined value, wherein this predefined value might be, for instance, -12 dB. This can lead to a further increased accuracy of determining the physiological parameter.

Preferentially, the first RF antenna and/or the second RF antenna have a width and a length which are each smaller than 30 cm. In particular, the dimensions of the first RF antenna and/or the second RF antenna can be such that each of the first and the second RF antenna can be arranged within a virtual sphere having a diameter of 30 cm. Thus, preferentially the RF antenna module, comprising the first and second RF antenna, is not too large, in order to allow the RF antenna module to be relatively easily integrated into the measurement device, which might be configured to be worn by the subject. In particular, the RF antenna module can be integrated into a holder of the measurement device for holding the RF antenna module. In a preferred embodiment, the first and/or the second RF antenna have a width and a length which are each within a range of 1 cm to 30 cm and further preferred within a range from 10 cm to 20 cm. Thus, in a preferred embodiment, the first and/or the second RF antenna are configured such that the respective antenna can be arranged within a virtual sphere having a diameter being equal to or smaller than 30 cm, but it cannot be arranged within a virtual sphere having a diameter being equal to 1 cm, and, in a further preferred embodiment, the first and/or the second RF antenna are configured such that the respective antenna can be arranged within a virtual sphere having a diameter being equal to or smaller than 20 cm, but it cannot be arranged within a virtual sphere having a diameter being equal to 10 cm. An RF antenna module with these dimensions is optimized for providing a motion signal being indicative of cardiac motion.

Moreover, preferentially the RF antenna module comprises at least one of the following: a dipole antenna, a loop antenna, also known as loop coil, or a microstrip antenna. In particular, at least one of the first RF antenna and the second RF antenna can be a dipole antenna, a loop antenna, or a microstrip antenna. In an embodiment, at least one of the first RF antenna and the second RF antenna is a dipole antenna. It has been found that a dipole antenna can provide a more complete and uniform coverage of the internal structure, which enables a more complete and uniform spatial sensitivity of determining the physiological parameter. This allows for an even more improved determination of the physiological parameter.

Hence, in a preferred embodiment, the RF antennas are dipole antennas. Since in this embodiment the RF antennas are dipole antennas, the electromagnetic phase profiles of the RF antennas can be symmetric, allowing the RF antennas to be used in an opposing configuration such that the electromagnetic phase profiles of the antennas cancel out each other to an even higher degree. The spatial sensitivity of determining the physiological parameter can be even more uniform in this case, which further improves the determination of the physiological parameter.

A dipole antenna can be an antenna comprising antenna sections. It shall be understood that the dipole antenna radiates or receives electromagnetic radiation as a whole, even if it comprises several, for instance, two, antenna sections. Preferably, the dipole antenna comprises two antenna sections. Each of the antenna sections can comprise one or more conductive elements. The one or more conductive elements can be formed, for instance, from elongated conductors. In particular if an antenna section comprises more than one conductive element, the conductive elements can be connected to each other either directly or via one or more electrical components. In an example, the dipole antenna can comprise two straight conductive elements, each of which is formed by a straight elongated conductor. In this example, the two straight conductive elements can be arranged in a straight line such that a gap remains between the two conductors. In this example, each one of the two straight conducting elements can be regarded as one antenna section and the position of the gap could also be referred to as center, or center point, of the dipole antenna of this example. More detailed descriptions of other exemplary embodiments of the antenna sections are provided below. Preferentially, the dipole antenna comprises two antenna sections and is configured such that in use the RF instrument is connected to the dipole antenna in between the two antenna sections. For instance, a matching circuit and/or an excitation source of the RF instrument can be connected to the dipole antenna in the center of the dipole antenna, so that the matching circuit and/or the excitation source of the RF instrument connects the two antenna sections. The matching circuit and the excitation source can be a well-known matching circuit and a well-known excitation source. In particular, the RF instrument can be connected to the dipole antenna via a feedline, wherein a first lead of the feedline is connected to the first antenna section and a second lead of the feedline is connected to the second antenna section. The point where the feedline is connected to the dipole antenna could also be referred to as feed port. The feedline could also be regarded a transmission line, wherein the term feedline is commonly used when RF power is mainly transmitted into the dipole antenna and the term transmission line is commonly used when an RF signal is mainly received from the dipole antenna. It shall be noted, however, that the feedline/transmission line can, in general, both be used for transmitting an RF power to the antenna and for receiving an RF signal from the antenna and the terms feedline and transmission line thus will be used interchangeably in the following.

Preferentially, the antenna sections have an elongated shape. A size of the elongated antenna section along the elongated dimension could be regarded as a length of the antenna section. In a preferred embodiment, the dipole antenna comprises two antenna sections, wherein the two antenna sections have an elongated shape and are arranged end to end such that the overall length of the dipole antenna, is at least equal to, in particular equal to, the sum of the length of the first antenna section and the length of the second antenna section. The overall length of the dipole antenna is preferentially half of the RF wavelength which corresponds to the operating frequency. The overall length of the dipole antenna can also be shorter or longer depending on the elements of the matching circuit. Also for amending the overall length of the dipole antenna a known matching circuit can be used. In particular, the matching circuit can comprise inductors and/or capacitors that tune the dipole antenna to the desired operating frequency.

Furthermore, an additional matching circuit can be used to match the dipole antenna to a cable, in particular a feedline cable, like a coaxial cable for a maximized transmission efficiency. This cable is preferentially the cable to the RF instrument being preferentially a vector network analyzer. This additional matching circuit can be, for instance, a lattice balun as disclosed, for example, in the article "Lumped and Distributed Lattice-type LC-Baluns" by W. Bakalski et al., 2002 IEEE MTT-S International Microwave Symposium Digest, DOI:10.1109/MWSYM.2002.1011595, which is herewith incorporated by reference.

The antenna sections can comprise conductive elements, for instance conductive elements comprising metal. The antenna sections can be galvanically isolated from each other. In particular, the antenna sections can be galvanically isolated from each other when they are not connected to a feedline or transmission line. When the dipole antenna is in use, the antenna sections can be connected, for instance, via electrical components connected to the feedline or transmission line. The antenna sections can also be connected, in particular galvanically connected, even without being connected to the feedline or transmission line. In particular, the antenna sections can be connected via electrical components. For instance, the antenna sections can be connected via one or more resistors, capacitors, inductors, leads, or a combination of the aforementioned.

The antenna sections can have the same shape, composition and/or electrical properties. The antenna sections can also differ, for instance, in shape, composition or electrical properties. The dipole antenna can be configured to produce a radiation pattern approximating that of an elementary electric dipole. For instance, the dipole antenna can be configured to support a line current distribution similar to that of a thin straight wire so energized that the current has a node only at each end.

An antenna section of the dipole antenna can comprise a conductive element in the form of an elongated conductor. Dimensions along the elongated dimension of the elongated conductor will be referred to as length in the following. For instance, the antenna section can comprise a conductive wire or rod, wherein the dimension along the elongated dimension of the wire or rod is referred to as length of the wire or rod. The elongated conductor can have a cross-sectional profile, i.e. a profile perpendicular to the elongated dimension, which is round, elliptical, rectangular or square. The elongated conductor can have a cross-sectional profile which is hollow or full. For instance, the elongated conductor can comprise a metal wire or rod with a cross-sectional profile which is round, elliptical, rectangular or square. The elongated conductor can be continuous. The continuous elongated conductor can comprise several conductor portions. The elongated conductor can have the same cross-sectional profile along the elongated dimension, in particular without being interrupted. The conductor portions of the elongated conductor can have different shapes. In an example, the antenna section comprises an elongated conductor which is straight. In another example, the antenna section comprises an elongated conductor which is bent in one or several positions. In this example, some conductor portions of the elongated conductor can be bent, while other conductor portions can be straight. The elongated conductor can have one or more cross-sectional profiles which differ along the elongated dimension.

The antenna section can also comprise two or more conductive elements, in particular in the form of elongated conductors. The two or more elongated conductors can differ in their cross-sectional profile. For instance, one elongated conductor can be formed by a straight metal wire or rod and another elongated conductor can be formed by a hollow metal cylinder. When the antenna section comprises two or more conductive elements, the conductive elements can be connected to each other via electrical components. In particular, the conductive elements can be connected to each other via one or more of the following: a resistor, a capacitor, an inductor, a lead or a combination of the aforementioned. It shall be noted that several conductive elements, together with electrical components connected to the conductive elements and/or connecting the conductive elements to each other, can form an antenna section of the dipole antenna.

In some embodiments it is preferred that the dipole antenna has higher order resonance frequencies at frequencies at which the length is equal to a positive integer multiple of the respective wavelength. In such embodiments, at least one of the antenna sections of the dipole antenna can comprise conductive elements in the form of elongated conductors, wherein the corresponding bandwidth, which might be measured as full width at half maximum of the reflection, can be increased by increasing the width of at least one of the elongated conductors. Thus, the width of the elongated conductors is preferentially increased, in order to make the dipole antenna better usable at a wide range of operating frequencies.

In an embodiment, the dipole antennas are folded. A dipole antenna which is folded comprises at least one portion of at least one antenna section which is not straight, but bent. Dipole antennas which are folded are more stable, in particular in view of variations of the spatial permittivity of material surrounding the dipole antennas. This allows for further improving the determination of the physiological parameter.

In a preferred embodiment, at least one end of the respective dipole antenna is folded. An end of a dipole antenna refers to an end portion of one of the antenna sections of the dipole antenna, wherein the end portion is the portion of the antenna section not directly connected to the feedline or transmission line. That an end of the respective dipole antenna is folded thus refers to an end portion of one of the antenna sections comprising a bent portion. When both ends to the respective dipole antenna are folded, the dipole antenna is even more stable, in particular in view of variations in the spatial permittivity of the material surrounding the dipole antenna.

In an example, the first and second RF antennas are both dipole antennas, each having two ends, wherein both ends to the respective dipole antenna are folded. In this example, both the first and the second RF antenna are more stable, in particular in view of variations in the spatial permittivity of the material surrounding the dipole antennas, and in view of variations in the spatial permittivity of the material in between the first and the second RF antenna. This further improves the determination of the physiological parameter.

A dipole antenna with a folded end can, for instance, comprise an antenna section that, starting from the feedline or transmission line, has a first straight portion, a bent portion and a second straight portion. The length of the folded end can then refer to the length of the second straight portion. In particular, when the bent portion of the antenna section is shaped to contribute significantly to the overall length of the dipole antenna, the length of the folded end can also refer to the sum of the length of the second straight portion and the bent portion. In a preferred embodiment, the length of the folded end is within a range from 40% to 49.5% of the overall length of the respective dipole antenna.

An antenna section with a folded end can, for instance, comprise a conductive element in the form of an elongated conductor, wherein a first and a second conductor portion of the elongated conductor are straight and wherein the first and the second straight conductor portions are connected to each other via a third portion of the elongated conductor which is bent. If the first and the second straight conductor portions are connected to each other via a bent conductor portion, the elongated conductor may be continuous, i.e., without an interruption between the first straight conductor portion, the bent conductor portion and the second straight conductor portion. Preferentially, the conductor portions are configured so that the first straight conductor portion and the second straight conductor portion are aligned parallel. The conductor portions can also be configured so that the first straight conductor portion and the second straight conductor portion form a 90 degree angle.

The antenna section with a folded end can also comprise several conductive elements in the form of elongated conductors, wherein a first and a second conductive element are straight and wherein the first straight conductive element and the second straight conductive element are connected to each other via an electrical component. In particular, the first and the second straight conductive elements can be connected to each other via any of the following: a resistor, a capacitor, an inductor, a lead or a combination of the aforementioned. That is, the antenna section can comprise straight portions which are formed by straight conductive elements, for instance in the form of straight, elongated conductors, and the bent portion of the antenna section can be formed by an electrical component such as a resistor, a capacitor, an inductor or a lead, or a combination of such electrical components. Preferentially, the conductive elements are configured so that the first straight conductive element and the second straight conductive element are aligned parallel. The conductive elements can also be configured so that the first straight conductive element and the second straight conductive element form a 90 degree angle.

In a preferred embodiment, the at least one end of the respective dipole antenna is folded such that in use, when the subject is located in between the opposing RF antennas, it is folded away from the subject. It was found that such an antenna is particularly stable in view of variations in the spatial permittivity due to, for instance, variations in the body composition and/or placement of the antenna. This allows for further improving the determination of the physiological parameter. That an end of a dipole antenna is folded away from the subject can refer to an end portion of one of the antenna sections of the dipole antenna being bent away from the subject. In particular, the end portion of the antenna section can be bent and arranged such that a first portion of the antenna section is positioned closer to the subject than a second portion of the antenna section, wherein the first portion of the antenna section is directly connected to the feedline or transmission line, wherein the second portion of the antenna section is connected to the feedline or transmission line via at least the first portion of the antenna section. A dipole antenna with an end folded away from the subject can, for instance, comprise an antenna section that, starting from the feedline or transmission line, has a first straight portion, a bent portion and a second straight portion. The bent portion of the antenna section can be configured such that in use, when the subject is located in between the opposing RF antennas, the first straight portion of the antenna section is positioned closer to the subject than the second straight portion of the antenna section. In an example, the first and second RF antennas are both dipole antennas, each having two ends, wherein both ends to the respective dipole antenna are folded such that in use, when the subject is located in between the opposing RF antennas, the ends are folded away from the subject.

In a preferred embodiment, the RF antennas are folded on a dielectric substrate. The dielectric substrate provides a defined surrounding for the RF antennas. The dielectric substrate can, furthermore, provide mechanical strength and isolate conducting portions of the RF antennas which are folded. The electrical characteristics, for instance the resonant frequency, of the RF antennas are thus less susceptible to change with variations in the surrounding, which further improves the determination of the physiological parameter. However, the first RF antenna and/or the second RF antenna can also be attached to or deployed on a dielectric substrate. In particular, the first RF antenna and/or the second RF antenna can also be attached to or deployed on a dielectric substrate, even if the respective antennas are not folded.

While in some embodiments described so far, the first RF antenna and/or the second RF antenna are a dipole antenna which comprises two antenna sections, it is also possible that the dipole antenna comprises only one antenna section, wherein then the one antenna section could also be referred to simply as antenna. It is also possible that the dipole antenna comprises more than two antenna sections. It should be noted that all descriptions of the antenna sections that were described above in connection with a dipole antenna with two antenna sections also apply to antenna sections of dipole antennas with only one antenna section or with more than two antenna sections.

In a preferred embodiment, the RF antenna module is configured such that the operating frequency of the RF antennas is lower than 1 GHz and further preferred lower than 600 MHz. It has been found that such operating frequencies enable uniform coverage of the structure within the subject with the RF signal, thus enabling a further improved determination of the physiological parameter. Preferentially, the RF antenna module is further configured such that the operating frequency of the RF antennas is higher than 30 MHz and more preferentially higher than 300 MHz. An operating frequency within the provided frequency range can be particularly advantageous if a single smaller structure, for instance the heart, should be monitored. In this case, the RF radiation does not cause power deposition throughout the whole body, but only in a region closer to the RF antennas such as, for instance, a heart region if the RF antennas are arranged on the subject's breast region.

In general, an RF antenna can be used both for transmission, i.e., conversion of an electrical signal into electromagnetic waves that propagate through space, and for reception, i.e., conversion of electromagnetic waves to an electrical signal. In a preferred embodiment, the RF antenna module is configured to use the first RF antenna for transmission and the second RF antenna for reception or vice versa. It has been found that using one of the first and second RF antenna for transmission and the other one of the first and second RF antenna for reception results in a particularly good determination of the physiological parameter. Additionally or alternatively, it is also possible to use one or both of the first RF antenna and the second RF antenna both for transmission and reception.

In a preferred embodiment, the RF antenna module is configured such that the electromagnetic phase profiles of the opposing RF antennas are symmetric in the sense that negative phases of an electromagnetic phase profile of the first RF antenna overlap with positive phases of an electromagnetic phase profile of the second RF antenna and vice versa. Since negative phases overlap with positive phases in this embodiment, the electromagnetic phase profiles of the antennas cancel out each other to an even higher degree and the spatial sensitivity of determining the physiological parameter can be even more uniform, which further improves the determination of the physiological parameter.

In an embodiment, at least one of the RF antennas is flexible to conform to the shape of the subject. With an RF antenna that conforms to the shape of the subject's body, there is less variation in the RF antenna's surroundings. For instance, since the RF antenna conforms to the shape of the subject's body, it can be at the same distance from the body surface everywhere and is not further away from the body surface in some places and less far away from the body surface in other places. This also reduces the variation in the RF antenna's surroundings from subject to subject, since the RF antenna conforms to the different body shapes. An RF antenna that conforms to the shape of the subject is also less likely to be unintentionally repositioned by subject movement. This results in more stable performance and further improves the determination of the physiological parameter.

In a preferred embodiment, at least one of the RF antennas comprises a capacitor and/or an inductor like a coil, which are connected in series by using conductors. The use of a capacitor and/or an inductor allows for adjusting the electrical properties of the RF antenna by adjusting the capacitance and inductance values, thereby improving the transmission and reception properties of the RF antenna for the respective use case.

In this embodiment, the RF antenna could also be seen as an interrupted conductor, wherein in one or several gaps in the conductor, which are caused by the interruption, a capacitor and/or an inductor is placed. In an example, an antenna section of a dipole antenna comprises two or more conductive elements, for instance in the form of elongated conductors. In this example, the conductive elements may be connected to the adjacent conductive element or the adjacent conductive element via a capacitor and/or an inductor.

Preferentially, at least one of the RF antennas comprises a capacitor and/or an inductor, wherein the capacitor has a capacity in a range from 2 to 10 pF and/or the inductor has an inductivity in a range from 5 to 15 nH. More preferentially, the capacitor has a capacity in a range from 4 to 8 pF and/or the inductor has an inductivity in a range from 8 to 12 nH. Preferentially, the overall capacitance of the respective RF antenna is in a range from 8 to 40 pF and/or the overall inductivity of the respective RF antenna is in a range from 10 to 30 nH. More preferentially, the overall capacitance of the respective RF antenna is in a range from 16 to 32 pF and/or the overall inductivity of the respective RF antenna is in a range from 16 to 24 nH.

In an embodiment, the respective RF antenna comprises several capacitors and several inductors. The use of several capacitors and/or inductors allows for an even better adjustment of the electrical properties of the RF antenna by adjusting the several capacitance and inductance values, thereby further improving the transmission and reception properties of the RF antenna for the respective use case.

In an example, the respective RF antenna comprises four capacitors and two inductors. In this example, the RF antenna may be a dipole antenna, wherein each of the antenna sections of the dipole antenna comprises two capacitors and one inductor. The two capacitors and one inductor of such an antenna section can connect four conductive elements such as, for instance, elongated conductors. As an example, a first conductive element can be connected to a second conductive element via one of the capacitors, the second conductive element can be connected to a third conductive element via one of the inductors and the third conductive element can be connected to a fourth conductive element via one of the capacitors. In an embodiment, the respective capacitors have a capacitance of 6 pF and the respective inductors have an inductivity of 10 nH.

In a preferred embodiment, the first and second RF antennas are constructed identically, wherein the RF instrument is configured to control the RF antennas such that the electromagnetic phase profiles of the first and second RF antennas at least partly cancel out each other. Since in this embodiment the RF antennas are constructed identically, the electromagnetic phase profiles of the RF antennas can be symmetric, allowing the RF antennas to be used in an opposing configuration such that the electromagnetic phase profiles of the antennas cancel out each other to an even higher degree. The spatial sensitivity of determining the physiological parameter can be even more uniform in this case, which further improves the determination of the physiological parameter.

In this example, the first RF antenna and the second RF antenna are constructed identically. However, the first RF antenna and the second RF antenna of the RF antenna module can also be constructed differently. The RF module can also comprise more than two RF antennas, wherein all of the RF antennas of the RF module can be constructed identically or differently or wherein some of the RF antennas of the RF module are constructed identically and others are constructed differently.

In a further aspect of the present invention a measurement device is presented, wherein the measurement device includes a) an RF antenna module comprising a first RF antenna and a second RF antenna and b) an RF instrument connected to the RF antenna module and configured to transmit RF power into the RF antenna module and to receive an RF signal from the RF antenna module, wherein the RF antenna module is configured to use the RF antennas in an opposing configuration such that electromagnetic phase profiles of the RF antennas at least partly cancel out each other, wherein in use a subject is located in between the opposing RF antennas.

In an embodiment the RF instrument, which in a preferred embodiment is a vector network analyzer, is configured to provide as the motion signal a complex signal. In particular, the RF instrument is configured to provide at least one of a) a complex reflection coefficient and b) a complex coupling coefficient as the motion signal. In a preferred embodiment, the processor is configured to identify a first subsignal of the complex signal having a distinct phase shift of, for example, 90 degrees with respect to a second subsignal of the complex signal and to determine the physiological parameter based on at least one of the identified subsignals, for example based on the first subsignal. Thus, the processor can be configured to process the motion signal such that a processed motion signal is obtained, i.e. for instance the identified first subsignal, and to determine the physiological parameter based on this processed motion signal. However, it is also possible that the measurement device, in particular the RF instrument of the measurement device, is configured to process the received RF signal and/or the complex signal and to provide a processed signal. For example, the RF instrument can be configured to identify the first subsignal of the complex signal having a distinct phase shift of, for example, 90 degrees with respect to the second subsignal of the complex signal and to provide, as the motion signal, the first subsignal or the second subsignal or a signal based on the first and/or second subsignal. In this case, the motion signal provided by the RF instrument could also be referred to as motion signal that has been processed or processed motion signal. In this example, the processor can be configured to determine the physiological parameter based on this processed motion signal as provided by the RF instrument of the measurement device.

It has been found that the complex signal can comprise contributions from at least two subsignals having a distinct phase shift relative to each other of, for instance, 90 degrees. In particular, one of these subsignals can be caused by cardiac motion and the other of these subsignals can be caused by respiratory motion. Thus, by identifying the first subsignal and using the identified first subsignal for determining a heart-related physiological parameter, the determination of the heart-related physiological parameter can be less influenced by respiratory motion, thereby allowing for an increased accuracy of determining the heart-related physiological parameter. Preferentially, the distinct phase is a predetermined phase, wherein the predetermined phase can be predetermined by, for example, a calibration procedure.

In another aspect of the present invention, a determination device for determining a physiological parameter of a subject based on a motion signal measured by the measurement device is presented, wherein the determination device comprises a model providing module configured to provide a model that provides, as an output, a physiological parameter if, as an input, a motion signal is provided, and a processor configured to determine the physiological parameter based on the provided model and the provided motion signal.

The model providing module can be configured to provide a model that provides, as an output, a physiological parameter if, as an input, the motion signal as provided by the RF instrument of the measurement device is provided. The motion signal as provided by the RF instrument of the measurement device can be, in particular, a processed motion signal which was obtained by processing the received RF signal and/or the complex signal. In this example the processor can be configured to determine the physiological parameter based on the provided model and the already processed motion signal provided by the RF instrument. In a preferred embodiment, the processor is configured to further process the motion signal provided by the RF instrument, wherein the motion signal provided by the RF instrument preferentially is the complex signal which can comprise contributions from at least two subsignals having a distinct phase shift relative to each other. In this case, the model providing module is preferentially configured to provide a model that provides, as an output, a physiological parameter if, as an input, a motion signal that is a processed motion signal determined by the processor is provided. The motion signal used by the processor as input to the model in order to determine the physiological parameter can thus differ from the motion signal provided by the RF instrument due to, for instance, processing steps performed by the processor.

The processor can be configured to determine the heart-related subsignal, i.e., the processed motion signal to be used for determining the heart-related subsignal, such that it has a maximum absolute or relative magnitude in a predefined expected frequency range in which the heart frequency is expected. This expected frequency range can be, for instance, 0.7 Hz to 1.5 Hz. In particular, the determination device can be configured to perform a phase rotation on the complex motion signal, which has been received from the measurement device and which is to be processed for generating the processed motion signal, such that the heart-related subsignal, i.e. the processed motion signal, is aligned with the real axis of a corresponding complex coordinate system. This can be performed by rotating the received complex motion signal in the complex coordinate system until the real part of the rotated complex motion signal has reached a maximum absolute or relative magnitude in the predefined expected frequency range in which the heart frequency is to be expected. For determining the magnitude of the subsignal in the predefined expected frequency range, the subsignal is preferentially transformed into the frequency domain by using, for instance, a Fourier transform. Thus, in an embodiment, a phase rotation is performed on the complex motion signal until the magnitude of the real part of the rotated complex motion signal has a maximum in the predefined expected frequency range, wherein, especially if the different subsignals related to different kinds of motion are separated by 90 degrees, the resulting subsignal, i.e. the resulting real part of the rotated complex total motion signal, is substantially only heart-related. The processor then can use this resulting subsignal, i.e. the processed motion signal in which respiratory influences have been reduced or even eliminated, for determining the heart-related physiological parameter with high accuracy.

The absolute magnitude of the subsignal in the predefined expected frequency range of, for instance, 0.7 Hz to 1.5 Hz can be, for instance, the maximum value within this frequency range or it can be the output of a function which has, as inputs, one or several magnitude values in the predefined frequency range. For example, the absolute magnitude of the subsignal in the predefined expected frequency range, which should be maximized, can be the average of the magnitude values in the predefined expected frequency range.

This absolute magnitude of the subsignal in the predefined expected frequency range can be directly used for finding the heart-related subsignal or it can be related to one or several magnitudes outside of the predefined expected frequency range for forming the relative magnitude of the predefined expected frequency range. For instance, the absolute magnitude of the subsignal in the predefined expected frequency range can be compared with the magnitude of the subsignal in another predefined unwanted frequency range that should be suppressed like a frequency range from 0.15 Hz to 0.25 Hz, if respiratory motion should be suppressed. For this comparison, an unwanted magnitude can be determined based on one or several magnitudes within the unwanted frequency range. For example, the unwanted magnitude can be the maximum magnitude within the unwanted frequency range or it can be the average of the magnitudes within the unwanted frequency range.

It is also possible that the absolute magnitude of the subsignal in the predefined expected frequency range is compared with another unwanted magnitude like a background magnitude. The background magnitude is the background with respect to the predefined expected frequency range. Thus, the background magnitude can be determined as the average of the magnitudes of the subsignal outside of the predefined expected frequency range.

The comparison for providing the relative magnitude of the expected frequency range can be carried out by subtracting the absolute magnitude of the subsignal in the predefined expected frequency range by a) the magnitude of the subsignal in the predefined unwanted frequency range, b) the background magnitude or c) a combination of the unwanted magnitude and the background magnitude. Also another comparison measure can be used like a division. Hence, the comparison for providing the relative magnitude of the expected frequency range can be carried out by dividing the absolute magnitude of the subsignal in the predefined expected frequency range by a) the magnitude of the subsignal in the predefined unwanted frequency range, b) the background magnitude or c) a combination of the unwanted magnitude and the background magnitude.

The processor can be configured to determine the subsignal, i.e. the processed motion signal, such that the comparison measure yields a maximum value. In particular, the total complex signal, i.e. the initially received motion signal, can be rotated in the complex coordinate system, i.e. the phase can be rotated, until the comparison has reached its maximum for the real part of the total complex signal, wherein this real part is the processed motion signal that is subsequently used by the processor for determining the physiological parameter.

In a preferred embodiment the processor is configured to apply a blind source separation technique, in order to generate a processed motion signal, and to use the processed motion signal and the provided model to determine the physiological parameter. Moreover, in an embodiment the model providing module is configured to provide as the model at least one of a linear regression model, a polynomial regression model and a Gaussian process regression model.

Thus, in an embodiment the processor is configured to apply a blind source separation technique such as independent component analysis (ICA) or principal component analysis (PCA) to the motion signal that has been received from the measurement device, in order to generate a processed motion signal, and to use the processed motion signal, which can also be regarded as being a subsignal of the initial motion signal, to determine the physiological parameter. It has been found that processing the motion signal by using second order blind identification (SOBI) yields even more accurate physiological parameters and therefore is preferred. Moreover, the processor can be configured to apply a frequency filtering to the motion signal and to use the resulting processed motion signal to determine the physiological parameter. The frequency filtering can be, for instance, a band-pass filtering, a low-pass filtering, a high-pass filtering or a Kalman filtering. This further processing of the measured signal finally allows for a further increased accuracy of determining the physiological parameter.

In particular, if the motion signal, which is received from the measurement device, is a complex signal, it in fact comprises two subsignals, for example, magnitude and phase or real part and imaginary part. The blind source separation technique like a principal component analysis (PCA) can be applied to these two subsignals. In particular, a vector can be defined with two vector elements, wherein the first vector element comprises one of the magnitude and phase and the second vector element comprises the other of the magnitude and phase. It is also possible that the first vector element comprises one of the real part and the imaginary part and the second vector element comprises the other of the real part and the imaginary part. The blind source separation technique can be applied to this vector, thereby generating a new vector, wherein the first vector element of the new vector is a first subsignal and the other vector element of the new vector is a second subsignal. These two subsignals are independent, uncorrelated or orthogonal with respect to each other due to the blind source separation technique. In order to determine which subsignal should be used for determining which physiological parameter, a frequency analysis can be performed on the two subsignals. In particular, the amplitude of the respective subsignal in a predefined expected frequency range, which is expected to be indicative for the respective physiological parameter to be determined, can be compared with the amplitude of the respective subsignal in one or several other frequency ranges, in particular, in all other frequency ranges. For instance, a Fourier transform can be carried out, in order to transform the respective subsignal into the frequency domain, wherein the value of the respective frequency spectrum in the expected frequency range can be compared with the value of the respective frequency spectrum outside of the expected frequency range, in particular, it can be compared with the value of the respective frequency spectrum in another unwanted frequency range which is indicative of unwanted motion to be suppressed like respiratory motion, if a heart-related parameter should be determined. The value of the respective frequency spectrum in the expected frequency range can also be compared with the average value of the respective background signal being defined as the average of the value over the whole frequency range excluding the expected frequency range. The comparison can be carried out by division, subtraction or another comparison measure. The subsignal, for which the value in the expected frequency range relative to the value in another unwanted frequency range being indicative of unwanted motion to be suppressed or relative to the background signal is largest, is selected to be the subsignal, i.e. the processed motion signal, which should be used by the processor for determining the physiological parameter. If the physiological parameter is a cardiac parameter, the expected frequency range can be, for instance, 0.7 Hz to 1.5 Hz and the unwanted frequency range can be, for instance, 0.15 Hz to 0.25 Hz. If the physiological parameter is a respiration-related parameter, the expected frequency range can be, for instance, 0.15 to 0.25 Hz and the unwanted frequency range can be, for instance, 0.7 Hz to 1.5 Hz.

It is also possible that the processor applies at least one of the blind source separation and the frequency filtering to the motion signal, which has been received from the RF instrument, for generating the processed motion signal to be used for determining the physiological parameter.

Preferentially, the model providing module is configured to provide a linear model as the model. It has been found that already a linear model can lead to a determination of a physiological parameter like a stroke volume or a heart rate with an increased accuracy such that relatively low computational efforts are required for, for instance, training the model and utilizing the model.

In an embodiment, the measurement device is configured to measure different motion signals for different frequencies, wherein the determination device is configured to determine the physiological parameter based on the motion signals measured for the different frequencies. In an embodiment, the different motion signals can be regarded as being a motion signal which depends on the frequency. In particular, in an embodiment the RF instrument is configured to transmit RF power into the RF antenna module with different frequencies, in order to provide the motion signal for the different frequencies, wherein the determination device is configured to determine the physiological parameter based on the motion signal provided for the different frequencies. Thus, measurements can be performed, wherein the operating frequency is changing in time. In particular, a frequency sweep can be performed. By measuring at multiple frequencies, both global and local motion effects can be distinguished, which can improve the accuracy of determining the physiological parameter. In an embodiment, different motion signals measured at different frequencies can be used to measure different physiological parameters, for example heart rate, stroke volume and tidal volume are measured at different frequencies. In another embodiment, motion signals at multiple frequencies can be combined, for example by averaging or a blind source separation technique like ICA, PCA or most preferentially SOBI, in order to determine a processed motion signal that can be used to determine the physiological parameter with a further improved accuracy.

The measurement device can be configured to measure different motion signals for different frequencies, wherein the determination device can be configured to combine the motion signals, which have been measured for the different frequencies, and to determine the physiological parameter based on the combined motion signals. Thus, the motion signals, which are received from the RF instrument, can be combined and thereby processed for determining a processed motion signal that can be used by the processor for determining a physiological parameter. The combination of the motion signals can be a linear combination. The linear combination can be determined by a blind source separation technique like a PCA or ICA. In particular, the processor can be configured to determine the physiological parameter based on the first principal component being, in this example, the processed motion signal. For instance, the model can provide a relation between the first principal component, i.e. the processed motion signal, and the physiological parameter, wherein the processor can be configured to determine the physiological parameter based on the first principal component and the relation. The relation and hence the model can be predetermined by calibration. It can be a linear relation. In an embodiment, the first principal component can be used for determining a heart-related physiological parameter like the stroke volume. The second principal component, which is a further processed motion signal, can be used, for instance, for determining a lung-related parameter.

In an embodiment, the motion signals obtained from the measurement device are complex and have been measured at different frequencies simultaneously with a gold standard measurement of the physiological property in a training phase. In particular, the gold standard measurement can be a measurement of the stroke volume by using transthoracic echo or magnetic resonance imaging (MRI). Since each received motion signal, which has been measured at the respective frequency, is complex, each motion signal in fact is formed by two subsignals like a phase subsignal and a magnitude subsignal or a real part subsignal and an imaginary part subsignal. The different subsignals, which have been measured at different frequencies, can be combined by using a blind source separation like PCA. Depending on the used blind source separation technique, the number of resulting separated subsignals can vary between two and the total number of initial subsignals. The new subsignals, i.e. the processed motion signals obtained by applying the blind source separation technique, are compared with the gold standard physiological parameter, wherein among the new subsignals the subsignal is selected, which correlates best with the gold standard physiological parameter. This can be carried out by using a comparison measure like a calculation of root-mean-square error or a calculation of a correlation, wherein the new subsignal having the lowest root-mean-square error or the highest correlation with the gold standard physiological parameter can be selected to be used in future physiological parameter determination procedures. Thus, this part of the training phase determines which new subsignal, for instance, in case of SOBI, which SOBI component, should be used for determining the physiological parameter. In a preferred embodiment, one of the first and second SOBI components, particularly the first SOBI component, is used for determining a heart-related physiological parameter or a lung-related physiological parameter.

The model which should provide the relation between the selected new subsignal, i.e. the processed motion signal, and the physiological parameter can also be determined in the training phase, wherein a linear regression model, a polynomial regression model or most preferentially a Gaussian process regression model can be used. In particular, the corresponding model can include one or several parameters which are modified such that, if the model is used together with the selected subsignal for determining the physiological parameter, this determined physiological parameter corresponds as good as possible to the gold standard physiological parameter. This training and also the other trainings described in this patent application can be done on a subject specific basis or on a group basis. After this training phase has been completed, the determination device can use the training result for determining the physiological parameter in future determinations. In particular, the same kind of combining the initially received motion signals, the same resulting new subsignal, i.e., the same processed motion signal, and the same adapted model can be used by the processor for determining the physiological parameter based on future RF measurements.

It has been found that a particularly accurate physiological parameter can be determined, if, as the processed motion signal, a SOBI component is used and as the model a Gaussian process regression model is used. In particular, one of the first and second SOBI components, particularly the first SOBI component, can be input into the Gaussian regression model for determining a heart-related physiological parameter or a lung-related physiological parameter.

In another embodiment, the subsignals of the acquired complex motion signals are directly used, i.e. they are not processed by using a blind source separation technique, for the comparison with the gold standard physiological parameter. For instance, the processor can be configured to determine which received subsignal of the received complex motion signals has the best correlation with the measured gold standard physiological parameter, wherein this best correlation subsignal can be used by the processor together with a corresponding model for determining the physiological parameter in future measurements. The correlation could be determined, for example, by regression analysis, Bland-Altman analysis, calculation of a root-mean-square error between the respective subsignal and the gold standard physiological parameter which, because of being measured over time, is also a signal, or by using another correlation measure. After this training, the processor can use the same selected subsignal in an actual measurement for determining the physiological parameter. Also in this embodiment, a corresponding model can be trained to provide, as an output, the physiological parameter, if, as an input, the selected type of subsignal, i.e. the selected motion signal, is provided. In a further embodiment, during a training phase, a subsignal of the received motion signals can be selected by comparing the absolute magnitude of the respective subsignal in a predefined expected frequency range or the relative magnitude of the respective subsignal in the predefined expected frequency range of the different subsignals with respect to each other, as described above. The subsignal, which has the highest absolute magnitude or relative magnitude within the predefined expected frequency range then can be selected, wherein this selected processed motion signal can be used together with the gold standard physiological parameter for training the model, which in future measurements can be used by the processor for determining the actual physiological parameter.

In order to measure the different motion signals for the different frequencies, the measurement device, in particular the RF instrument and the RF antenna module comprising the first RF antenna and the second RF antenna, can be configured to be operated at multiple frequencies in a frequency sweep. The sequentially obtained signals at different frequencies have different penetration depths and result in motion signals that can be acquired with the same sensor, i.e., with the same RF instrument and the same RF antenna module. By combining the signals acquired at different frequencies, the accuracy of the measurement and sensitivity to breathing and bulk motion related artifacts can be minimized. Thus, for instance, the heart-related physiological parameter can be determined even more accurately.

In a further aspect of the present invention, an RF antenna is presented, wherein the RF antenna is configured to be used as first RF antenna or second RF antenna of the RF antenna module of the measurement device as defined by claim 14.

In another aspect of the present invention, the use of an RF antenna as first RF antenna or second RF antenna of the RF antenna module of the measurement device as defined by claim 14 is presented.

In another aspect of the present invention, a set of a first RF antenna and a second RF antenna is presented, wherein the RF antennas are configured to be used in an opposing configuration such that electromagnetic phase profiles of the RF antennas at least partly cancel out each other, wherein in use a subject is located in between the opposing RF antennas. For instance, the set of RF antennas can be configured to be used as first RF antenna and second RF antenna of the RF antenna module of the measurement device as defined by claim 14.

In a further aspect of the present invention a training system for training a model to be used by the system for determining the physiological parameter of the subject is presented, wherein the training system comprises:
- a training physiological parameter measurement device for measuring a training physiological parameter of a subject,
- a model providing module configured to provide an adaptable model to be trained, wherein the model provides, as an output, a physiological parameter if, as an input, a motion signal is provided,
- an RF antenna module comprising a first RF antenna and a second RF antenna and an RF instrument connected to the RF antenna module and configured to transmit RF power into the RF antenna module, to receive an RF signal from the RF antenna module and to provide a motion signal that is related to a mechanical movement of a structure within the subject based on the received RF signal, wherein the RF antenna module is configured to use the RF antennas in an opposing configuration such that electromagnetic phase profiles of the RF antennas at least partly cancel out each other, wherein in use the subject is located in between the opposing RF antennas, and
- a training module configured to a) determine a physiological parameter of the subject based on the model to be trained and a motion signal provided by the RF instrument and the RF antenna module and b) modify the model such that a deviation between the determined physiological parameter and the training physiological parameter is reduced.

The training physiological parameter measurement device and the RF antenna module are preferentially configured such that the training physiological parameter and the motion signal can be determined simultaneously. In an embodiment, the training physiological parameter measurement device is configured to use the RF antenna module for measuring the training physiological parameter of the subject. This allows to train the model and finally determine the physiological parameter with an even further increased accuracy, because the same RF antenna module can be used for determining the physiological parameter of the subject based on the model and the provided motion signal and for determining the training physiological parameter. In general, however, the training physiological parameter measurement device can be configured to determine the training physiological parameter of the subject independently of the RF antenna module.

In a further aspect of the present invention, a method for controlling the measurement device as defined by claim 14 is presented, wherein the method comprises controlling the RF antenna module of the measurement device such that the electromagnetic phase profiles of the RF antennas of the RF antenna module at least partly cancel out each other, wherein in use a subject is located in between the opposing RF antennas.

In another aspect of the present invention a method for determining a physiological parameter of a subject is presented, wherein the method comprises:
- providing a motion signal that is related to a mechanical movement of a structure within the subject by using an RF instrument and an RF antenna module of a measurement device as defined by claim 14,
- providing a model that provides, as an output, a physiological parameter if, as an input, a motion signal is provided, by a model providing module, and
- determining the physiological parameter based on the provided model and the provided motion signal by a processor.

In a further aspect of the present invention a training method for training a model to be used by the system for determining a physiological parameter of a subject, particularly as defined by any of claims 1 to 13, is presented, wherein the training method comprises:
- providing a model to be trained by a model providing module, wherein the model provides, as an output, a physiological parameter if, as an input, a motion signal is provided,
- measuring a training physiological parameter of a subject by a training physiological parameter measurement device and providing a motion signal that is related to a mechanical movement of an organ within a subject by using an RF antenna module comprising a first RF antenna and a second RF antenna and an RF instrument connected to the RF antenna module and configured to transmit RF power into the RF antenna module, to receive an RF signal from the RF antenna module and to provide a motion signal that is related to a mechanical movement of a structure within the subject based on the received RF signal, wherein the RF antenna module is configured to use the RF antennas in an opposing configuration such that electromagnetic phase profiles of the RF antennas at least partly cancel out each other, wherein in use the subject is located in between the opposing RF antennas, and
- determining a physiological parameter of the subject based on the model to be trained and the motion signal provided by the RF instrument and the RF antenna module and modifying the model such that a deviation between the determined physiological parameter and the training physiological parameter is reduced by a training module.

In a further aspect of the present invention, a computer program for controlling the system for determining a physiological parameter of a subject as defined by claim 1 is presented, wherein the computer program comprises program code means for causing the measurement device to control the RF antenna module such that the electromagnetic phase profiles of the RF antennas of the RF antenna module at least partly cancel out each other, wherein in use a subject is located in between the opposing RF antennas. Moreover, preferentially, this computer program or another computer program comprises program code means for causing the determination device to determine the physiological parameter based on a provided model, which provides, as an output, a physiological parameter if, as an input, a motion signal is provided. The at least one computer program can be configured to run on the RF instrument and/or on the processor of the determination device. The at least one computer program can also be configured to run on a controller of the measurement device and/or on a controller of the determination device. The at least one computer program can also be configured to run on a controller of the system for determining the physiological parameter of the subject. For example, the at least one computer program can be configured to run on a controller of the system for determining the physiological parameter of the subject, wherein the controller of the system is configured to control the measurement device, in particular the RF instrument of the measurement device, and the determination device.

In a further aspect of the present invention, a computer program for controlling the measurement device as defined by claim 14 is presented, wherein the computer program comprises program code means for causing the measurement device to control the RF antenna module such that the electromagnetic phase profiles of the RF antennas of the RF antenna module at least partly cancel out each other, wherein in use a subject is located in between the opposing RF antennas.

The computer program can be configured to run on the RF instrument or on a controller of the measurement device, which is configured to control the different components of the measurement device.

Preferentially, the computer program for controlling the measurement device further comprises program code means for causing the measurement device to provide a motion signal that is related to a mechanical movement of a structure within the subject by using an RF instrument and an RF antenna module of the measurement device.

In a further aspect of the present invention a computer program for controlling a determination device for determining a physiological parameter is presented, wherein the computer program comprises program code means for causing the determination device to determine the physiological parameter based on a provided model, which provides, as an output, a physiological parameter if, as an input, a motion signal is provided, and a motion signal which has been measured by a measurement device as defined by claim 14. The computer program can be configured to run on the processor of the determination device or on a controller of the determination device, which is configured to control the different components of the determination device.

In another aspect of the present invention a computer program for controlling the training system is presented, wherein the computer program comprises program code means for causing the training system to carry out the steps of the training method, when the computer program is run on a computer controlling the training system. The computer program can be configured to run on one or several components of the training system or on a controller of the training system, which is configured to control the different components of the training system.

In another aspect of the present invention, a further system for determining a physiological parameter of a subject is presented, the system comprising:
- a measurement device including a) an RF antenna module comprising a first RF antenna and a second RF antenna and b) an RF instrument connected to the RF antenna module and configured to transmit RF power into the RF antenna module, to receive an RF signal from the RF antenna module and to provide a motion signal that is related to a mechanical movement of a structure within the subject based on the received RF signal,
- a determination device configured to determine the physiological parameter based on the provided motion signal, wherein the determination device comprises a model providing module configured to provide a model that provides, as an output, a physiological parameter if, as an input, a motion signal is provided, and a processor configured to determine the physiological parameter based on the provided model and the provided motion signal,
wherein the RF antenna module is configured to use the RF antennas in an opposing configuration, wherein in use the subject is located in between the opposing RF antennas.

In an embodiment, the RF antennas are dipole antennas. Preferably, the dipole antennas are folded. Preferably, at least one end of the respective dipole antenna is folded. When at least one end of at least one dipole antenna is folded, the length of the folded end is preferentially within a range from 40% to 49.5% of the overall length of the respective dipole antenna. Preferably, the at least one end of the respective dipole antenna is folded such that in use, when the subject is located in between the opposing RF antennas, it is folded away from the subject. The RF antennas are preferentially folded on a dielectric substrate.

In an embodiment, the RF antenna module is configured such that the operating frequency of the RF antennas is lower than 1 GHz and further preferred lower than 600 MHz.

In an embodiment, the RF antenna module is configured to use the first RF antenna for transmission and the second RF antenna for reception or vice versa.

In an embodiment, at least one of the RF antennas comprises a capacitor and/or an inductor like a coil, which are connected in series by using conductors. Preferably, the capacitor has a capacity in a range from 2 to 10 pF and/or the inductor has an inductivity in a range from 5 to 15 nH. Preferably, the respective RF antenna comprises several capacitors and several inductors.

In an embodiment, the first RF antenna and the second RF antenna are constructed identically. Preferably, the RF antenna module is configured such that electromagnetic phase profiles of the opposing RF antennas are identical.

It shall be understood that the system for determining a physiological parameter of a subject of claim 1, the measurement device of claim 14, the determination device, the RF antenna of the measurement device, the use of the RF antenna, the set of a first RF antenna and a second RF antenna of claim 15, the training system, the method for determining a physiological parameter of a subject, the method of controlling the measurement device, the training method, the computer program for controlling a measurement device, the computer program for controlling a determination device for determining a physiological parameter, the computer program for controlling a training system, and the second system for determining a physiological parameter of a subject have similar and/or identical preferred embodiments, particularly as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

- Fig. 1: shows schematically and exemplarily an embodiment of a system for determining a physiological parameter of a subject,
- Fig. 2a: shows schematically and exemplarily a measurement device of the system shown in Fig. 1, wherein the measurement device is configured to be worn by the subject, and wherein a subject wearing the measurement device is shown from the front,
- Fig. 2b: shows schematically and exemplarily the measurement device and the subject of Fig. 2, wherein the subject is shown from behind,
- Fig. 3: shows schematically and exemplarily an embodiment of a determination device of the system shown in Fig. 1, wherein the determination device is configured to determine the physiological parameter based on a motion signal provided by the measurement device shown in Fig. 2,
- Fig. 4a: shows schematically and exemplarily an embodiment of a dipole antenna which is not folded,
- Fig. 4b: shows schematically and exemplarily an embodiment of a dipole antenna which is folded,
- Fig. 4c: shows schematically and exemplarily another embodiment of a dipole antenna which is folded,
- Fig. 4d: shows schematically and exemplarily a further embodiment of a dipole antenna which is folded,
- Fig. 5a: shows schematically and exemplarily a subject positioned between two dipole antennas with folded ends,
- Fig. 5b: shows schematically and exemplarily a subject positioned between two dipole antennas with folded ends,
- Fig. 6: shows schematically and exemplarily a thorax of a subject and illustrates schematically precordial lead positions V1 to V6 in electrocardiography,
- Fig. 7: exemplarily and schematically illustrates the electric field term, permittivity term, and differential reflection coefficient for a single loop antenna placed on the thorax of a subject,
- Fig. 8: shows exemplarily the simulated spatial sensitivity of RF sensing on a human model of a subject,
- Fig. 9: shows exemplarily simulation results of the reflection coefficient S*₁₁* for different RF antenna configurations and variations in placement,
- Fig. 10a: shows schematically and exemplarily a general antenna design used for an optimization process for a dipole antenna,
- Fig. 10b: shows schematically and exemplarily a reference antenna design used for an optimization process for a dipole antenna and simulations results for the reflection coefficient obtained with the reference design,
- Fig. 10c: shows schematically and exemplarily a dipole antenna design with lumped elements used for an optimization process for a dipole antenna and simulations results for the reflection coefficient obtained with the optimized dipole antenna,
- Fig. 11: shows exemplarily the simulated spatial sensitivity of RF sensing using the dipole antenna shown in Fig. 10c on a human model of a subject,
- Fig. 12: shows exemplarily and schematically an experimental realization of the dipole antenna shown in Fig. 10c and experimental results for the reflection coefficient *S₁₁* and the coupling coefficient *S₁₂* obtained using such dipole antennas for RF sensing,
- Fig. 13: shows exemplary experimental results of a reproducibility test performed with the antennas of Fig. 12,
- Fig. 14: shows schematically and exemplarily a training system for training the model to be provided by the model providing module,
- Fig. 15a: shows schematically and exemplarily a complex signal provided by the measurement device,
- Fig. 15b: shows schematically and exemplarily the signal shown in Fig. 15a after a phase rotation has been carried out,
- Fig. 15c: shows schematically and exemplarily the signal shown in Fig. 15b after a band-pass filter has been applied,
- Fig. 16: shows a flowchart exemplarily illustrating an embodiment of a method for determining a physiological parameter of a subject, and
- Fig. 17: shows a flowchart exemplarily illustrating an embodiment of a training method for training a model to be used by the system for determining the physiological parameter of the subject.

### DETAILED DESCRIPTION OF EMBODIMENTS

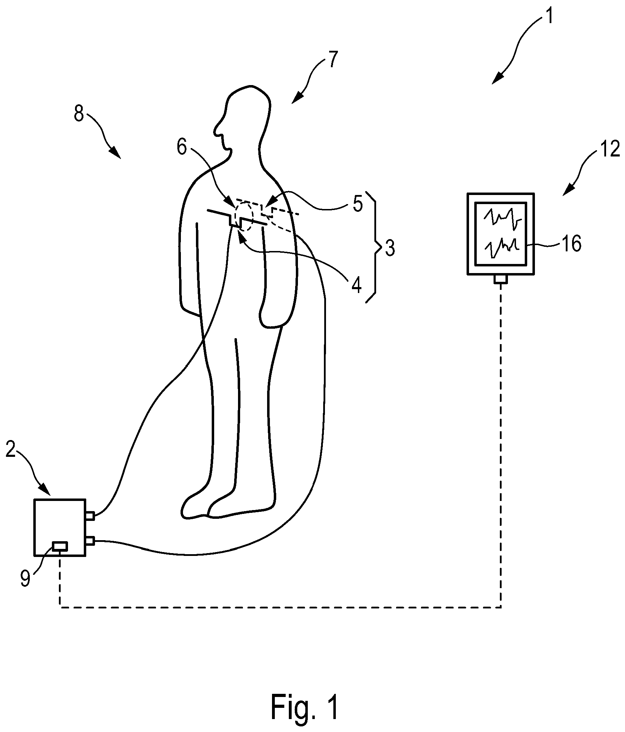

Fig. 1 illustrates schematically and exemplarily an embodiment of a system for determining a physiological parameter of a subject. In this embodiment the system 1 is configured to determine the stroke volume of the heart 6 within the subject 7 as the physiological parameter. However, the system also can be configured to determine another heart-related physiological parameter like the heart rate, or a lung-related physiological parameter like the respiratory rate or the tidal volume.

The system 1 comprises a measurement device 8 of which in Fig. 1 only the first RF antenna 4 and the second RF antenna 5 of an RF antenna module 3 and an RF instrument 2 are shown. The RF instrument 2 can, in particular, comprise a vector network analyzer. The RF antennas 4, 5 are connected to the RF instrument 2, wherein the RF instrument 2 and the RF antenna module 3, in particular the RF antennas 4, 5, are configured to provide motion signals that are related to a mechanical movement of the heart 6 within the subject 7.

In the exemplary embodiment illustrated in Fig. 1, the first RF antenna 4 is positioned on the front of the thorax of the subject 7, while the second RF antenna 5 is positioned on the back of the thorax. The subject 7 is thus located in between the first RF antenna 4 and the second RF antenna 5. The RF antenna module 3 is configured to use the RF antennas 4, 5 in an opposing configuration such that electromagnetic phase profiles of the RF antennas 4, 5 at least partly cancel out each other. The first RF antenna 4 and the second RF antenna 5 as shown in Fig. 1 are dipole antennas, positioned and aligned so that the longitudinal axes of the two antennas 4, 5 are parallel. Each one of the RF antennas 4, 5 thus emits a nearly linearly polarized electric field, wherein the electromagnetic phase profiles of the two RF antennas 4, 5 are symmetric and cancel out each other to a high degree. As schematically shown in Fig. 1, the first RF antenna and the second RF antenna preferentially have an elongated shape, wherein the antennas are preferentially arranged with a horizontal alignment, so that they can be placed in a strap or belt. Furthermore, it is preferred that the first RF antenna and the second RF antenna are aligned parallel to each other. A deviation from a parallel alignment can lead to an attenuation of the received signal. In a preferred embodiment, the two RF antennas 4, 5 are identical dipole antennas so that the electromagnetic phase profiles of the two RF antennas 4, 5 cancel out each other to a particularly high degree. In general, however, the RF antenna module of system 1 could also comprise a different kind of antenna as a first and/or second RF antenna. For instance, the first RF antenna and/or the second RF antenna could, in another embodiment, be a loop antenna or a microstrip antenna, as long as the electromagnetic phase profiles of the first RF antenna and the second RF antenna can be configured to at least partly cancel out each other.

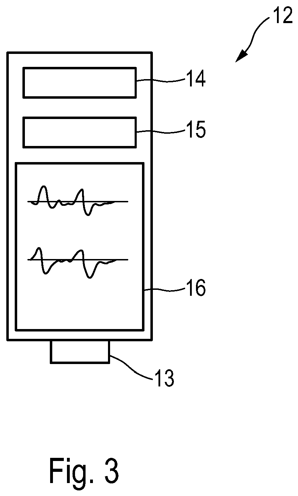

The system 1 further comprises a determination device 12, which is configured to determine the physiological parameter based on the provided motion signal. The determination device can be, for instance, a mobile device such as a smartphone, a tablet computer, or a laptop. An exemplary embodiment of a determination device 12 is shown schematically and with more details in Fig. 3. The determination device 12 comprises a model providing module 14 configured to provide a model that provides, as an output, a physiological parameter, if, as an input, a motion signal is provided. The determination device 12 further comprises a processor 15 configured to determine the physiological parameter, i.e., the stroke volume in this embodiment, based on the provided model and the provided motion signal. The provided motion signal is, in particular, the motion signal provided by the RF antenna module 3 and the RF instrument 2. The measurement device 8 can further comprise a transmitter for transmitting the provided motion signals to the determination device 12. In the exemplary embodiment shown in Fig. 1, a transmitter 9 is integrated in the RF instrument 2, for instance as part of a vector network analyzer. The transmission between the measurement device 8 and the determination device 12, particularly between the RF instrument 2 of the measurement device 8 and the determination device 12, is preferentially a wireless transmission like a Bluetooth transmission or any other wireless transmission. The determination device 12 exemplarily comprises a receiver 13 for receiving the motion signals provided by the measurement device 8.

The determination device 12 shown in Fig. 1 exemplarily also comprises a display 16 for displaying, for instance, the provided motion signal, a signal obtained by processing the provided motion signal, and/or the physiological parameter. Additionally or alternatively, the determination device 12 can comprise another output unit like a connector for connecting a display or establishing a data connection to another device.

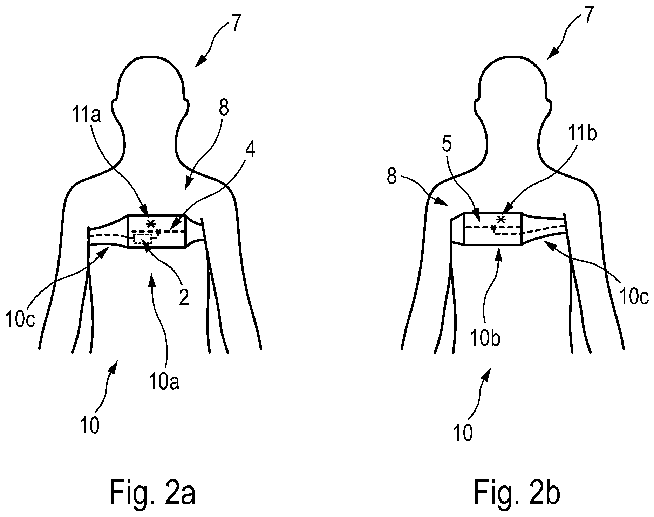

It should be noted that Fig. 1 just shows a schematic illustration of the system 1 for determining a physiological parameter of the subject 7, whereas in an actual measurement device, for instance, the RF instrument 2 can also be arranged on the body of the subject 7 and not somewhere in the air as shown in Fig. 1. For instance, as illustrated in Figs. 2a and 2b, the measurement device 8 can, in an embodiment, include a wearable holder 10. A first part 10a of the wearable holder 10 houses the first RF antenna 4 and the RF instrument 2, as indicated schematically and exemplarily in Fig. 2a by dashed structures, while the second part 10b of the wearable holder 10 houses the second RF antenna 5. The wearable holder 10 can, furthermore, comprise a strap 10c, which guides a cable connecting the RF instrument 2 with the second RF antenna 5. The wearable holder 10 thus integrates some or all of the components of the measurement device 8.

Moreover, the first part 10a of the wearable holder 10 can comprise a first visible marker 11a and the second part 10b of the wearable holder 10 can comprise a second visible marker 11b assigned to anatomical features of the subject 7. The wearable holder 10 can be configured to be worn such that the visible markers 11a, 11b are arranged at positions on the subject 7 at which the assigned anatomical features are located. In this embodiment, one anatomical feature can be the sternum at the height of the nipples, wherein, if the wearable holder 10 is worn correctly, the first visible marker 11a coincides with the sternum at the height of the nipples. In this embodiment, another anatomical feature can be the vertical center of the right edge of the left shoulder blade, wherein, if the wearable holder 10 is worn correctly, the second visible marker 11b coincides with the vertical center of the right edge of the left shoulder blade. In particular, the visible marker 11a can be configured such that when the visible marker 11a is positioned at the assigned anatomical feature, the center point of the first RF antenna 4 is located at one of the precordial lead positions in electrocardiography. The precordial lead positions in electrocardiography can be defined as follows:
- V1 - Fourth intercostal space (ICS), right of the sternum
- V2 - Fourth ICS, left of the sternum
- V3 - Midway between V2 and V4
- V4 - Fifth ICS, midclavicular line (just below the left nipple in men)
- V5 - Fifth ICS, anterior axillary line (in line with V4 but further left)
- V6 - Fifth ICS, midaxillary line (in line with V4 and V5, further left).

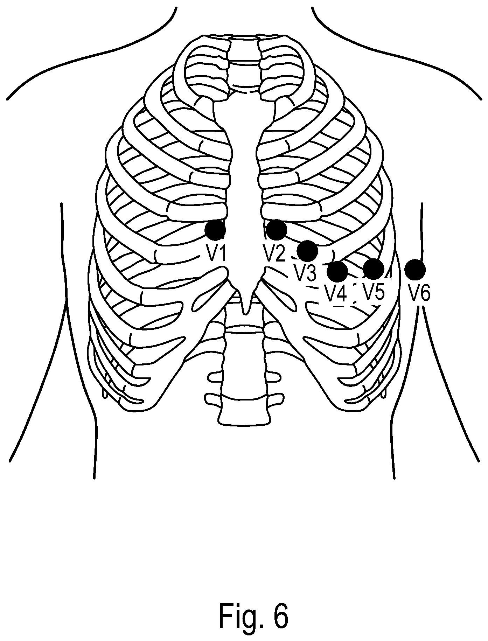

Fig. 6 schematically and exemplarily illustrates the precordial lead positions in electrocardiography accordingly. The visible markers 11a and 11b are preferably configured such that, when the visible markers 11a, 11b are positioned at the respective assigned anatomical feature, the center point of the second RF antenna 5, when seen in the coronal or frontal plane, is placed within a 10 cm diameter circle around the center point of the first RF antenna 4. More preferably, the visible markers 11a and 11b are configured such that, when the visible markers 11a, 11b are positioned at the respective assigned anatomical feature, the center point of the second RF antenna 5, when seen in the coronal or frontal plane, is placed within a 2 cm diameter circle around the center point of the first RF antenna 4. The visible markers 11a and 11b are preferably configured such that, when the visible markers 11a, 11b are positioned at the respective assigned anatomical feature, a virtual line from the center point of the first RF antenna 4 to the center point of the second RF antenna 5 intersects the heart 6 of the subject 7. For instance, the center point of the first RF antenna 4 can be positioned left to the sternum, according to precordial electrocardiography lead placement V2. With this placement, the first RF antenna 4 is positioned directly above the heart 6, thereby allowing to determine the heart-related physiological parameter even more accurately. The center point of the second RF antenna 5 can be positioned on the back side of the thorax above the heart 6 as well, so that a virtual line from the center point of the first RF antenna 4 to the center point of the second RF antenna 5 intersects the heart 6. With this placement, the heart 6 is positioned in between the first RF antenna 4 and the second RF antenna 5, thereby allowing to further increase the accuracy of determining the heart-related physiological parameter.

Especially when the RF antennas and the RF instrument 2 of the measurement device 8 are integrated into a wearable holder, the RF antennas of the RF antenna module 3 are preferentially lightweight, in particular with a weight of each RF antenna being smaller than 30 g. The RF antennas of the measurement device 8 are preferably made from flexible material so that they can align closely with the body surface and maintain the same distance to the subject everywhere. The wearable holder 10 can also be part of or integrated into clothing. In an embodiment, the wearable holder houses the RF antennas, in particular the first and second RF antennas 4, 5, but not the RF instrument 2. In this embodiment the RF instrument 2 can be a stationary device, a mobile or handheld device or it can be integrated into another wearable holder.

The RF instrument 2 is configured to provide a motion signal that is related to a mechanical movement of the structure 6 within the subject 7 based on the received RF signal. In use, the RF antenna module 3, i.e., at least one of the first RF antenna 4 and the second RF antenna 5, transmits RF electromagnetic radiation into the surrounding and at least one of the first RF antenna 4 and the second RF antenna 5 receive electromagnetic radiation from the surrounding. An RF antenna has a measurable complex impedance which quantifies the relation between the complex current and voltage at a feed port of the respective RF antenna. The antenna impedance changes in phase and magnitude when the surrounding of the respective RF antenna changes. This happens when an RF antenna is positioned on the body and there is motion of a structure inside the body. In this exemplary embodiment, the first RF antenna 4 and the second RF antenna 5 are positioned on the body of the subject 7, particularly in the vicinity of the heart 6 and lungs. The impedance of the first and second RF antennas 4, 5 as well as the coupling between the first RF antenna 4 and the second RF antenna 5 change when there is motion of the heart and lungs. This effect can be utilized to measure internal physiological motion with RF antennas. In an embodiment, the RF instrument 2 comprises a vector network analyzer which is configured to provide as a motion signal a complex signal like a complex reflection coefficient signal or a complex coupling coefficient signal. Since the RF antenna module 3, in this embodiment, comprises two RF antennas 4, 5, the vector network analyzer provides two complex signals to the determination device 12. The determination device 12 is configured to determine the physiological parameter, for instance, the stroke volume in this embodiment, based on the two complex signals provided by the vector network analyzer and the model provided by the model providing module. Further details regarding the determination of the physiological parameter will be described below.

In the described embodiment, the region of interest is the thorax and, more particularly, the heart 6 of the subject 7. Preferentially, one of the first and second RF antennas 4, 5 is used as a transmitting antenna and the other one of the first and second RF antennas 4, 5 is used as a receiving antenna. By placing the RF antennas 4, 5 on opposite sides of the region of interest, the electromagnetic phase profile of the transmitting and receiving antenna cancel out, leading to a spatial sensitivity with uniform sensitivity over the whole region of interest, i.e., in this embodiment the thorax and, in particular, the heart 6 of the subject 7. The RF instrument 2 and the RF antenna module 3 are configured such that the operating frequency of the RF antennas 4, 5 is lower than 1 GHz and higher than 30 MHz. In particular, the RF instrument 2 and the RF antenna module 3 are configured to be operated in a frequency range from 300 MHz to 600 MHz. In a preferred embodiment, the operating frequency of the RF instrument 2 and the RF antenna module 3 is 433 MHz. These operating frequencies enable uniform coverage of the heart or at least a single heart chamber. By providing a uniform coverage over the heart, a more stable and reproducible measurement of the physiological parameter is possible, considering both inter- and intra-subject variation

It has been found that RF antennas like dipole antennas comprising only two elongated straight conductors can be affected in performance by variations in loading, e.g. variations in body composition, shape, size and antenna placement on the body. While measurable changes in the antenna impedance, reflection coefficients and coupling coefficients due to changes in body composition caused by physiological motion are desired, these effects are preferably not so strong that they affect antenna performance. For instance, it was found that large changes in the resonant frequency of the antennas can have a strong effect on antenna performance and distort the phase of the output motion signal. It was further found that antenna performance can deteriorate in subjects with irregular body composition. These effects can result in measurement instability.

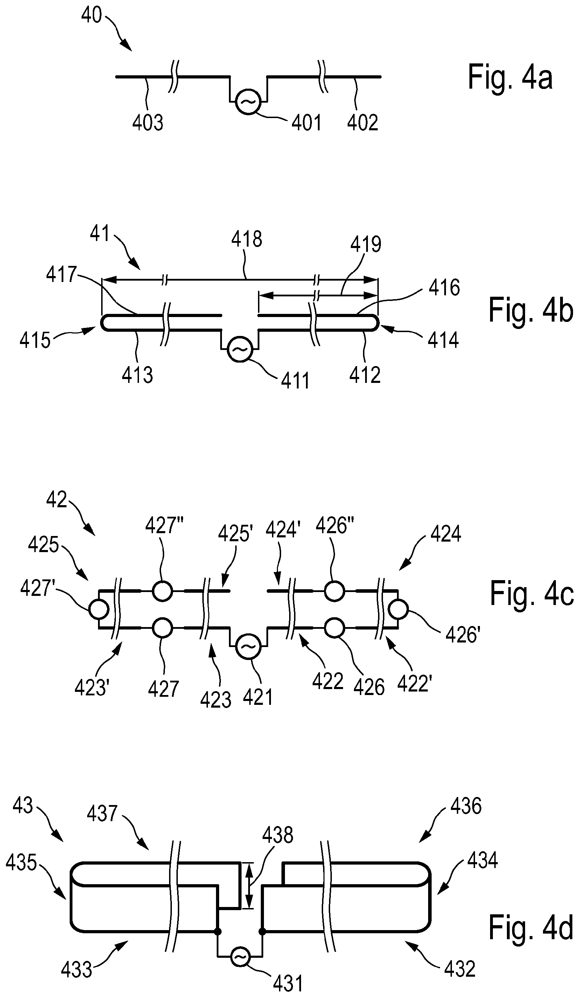

Several dipole antenna designs have been found by the inventors which are more stable against performance deterioration due to load variation. In a preferred embodiment of the system 1 for determining a physiological parameter of a subject, the first RF antenna 4 and/or the second RF antenna 5 are dipole antennas which are folded. Preferentially, at least one end of the respective dipole antenna is folded. Figs. 4b to 4d schematically and exemplarily show dipole antennas which are folded, while Fig. 4a schematically and exemplarily shows a dipole antenna which is not folded for comparison. The dipole antenna 40 in Fig. 4a comprises two conductive elements in the form of elongated straight conductors 402, 403, which are connected via an excitation source 401, which could be the excitation source of the RF instrument. The two elongated straight conductors 402, 403 could also be referred to as antenna sections. It shall be understood that Fig. 4a is a schematic illustration and therefore omits several components, such as matching circuits, which might be required in reality to operate the dipole antenna 40. For the purpose of understanding the operation of the dipole antenna 40, these components may be thought of as being integrated in the excitation source 401. The same applies to the respective excitation sources 411, 421, 431 in Figs. 4b, 4c and 4d, respectively. It shall also be understood that the dipole antennas in Figs. 4a to 4d are not shown to scale and are shown with break lines for the sake of clarity.

Fig. 4b schematically and exemplarily shows a dipole antenna 41 with folded ends. In this case, two conductive elements in the form of elongated conductors are connected via the excitation source 411. Each one of the two conductive elements could also be referred to as an antenna section of the dipole antenna 41. The first elongated conductor can be described as comprising several conductor portions, wherein two conductor portions 412, 416 of the first elongated conductor are straight, while one conductor portion 414 is bent. The second elongated conductor can also be described as comprising several conductor portions, wherein two conductor portions 413, 417 of the second elongated conductor are straight, while one conductor portion 415 is bent. The bent conductor portions 414, 415 thus cause the ends of the elongated conductors to be folded. The term folded end thus refers to the end of the elongated conductor which is not directly connected to the excitation source 411. The dipole antenna 41 has an overall length 418, wherein the overall length 418 is preferably much larger than a gap between the two antenna sections formed by the conductor portions 412, 414, 416 and 413, 415, 417, respectively, and/or a distance between the two straight conductor portions 412 and 416 or between the two straight conductor portions 413 and 417. The length 419 of the folded end is preferably within a range from 40% to 49.5% of the overall length 418 of the respective dipole antenna. The length 419 of the folded end is preferably similar or equal to the length of the straight conductor portion 412 and/or the straight conductor portion 416. However, the length 419 can in general also be different from the length of the straight conductor portion 416 and/or, in particular, different from the length of the straight conductor portion 412. For instance, if the bent conductor portion 414 is shaped to contribute significantly to the overall length 418 of the dipole antenna 41, the length 419 of the folded end can also refer to the sum of the length of the straight conductor portion 416 and an additional length due to the bent conductor portion 414. In an embodiment, the sum of the length of the folded ends of the dipole antenna 41 is equal to the overall length 418 of the dipole antenna 41. In this embodiment, the two straight conductor portions 416 and 417 can be connected. In particular, the two straight conductor portions 416 and 417 can form one straight elongated conductor in this embodiment. For the dipole antenna which is exemplarily shown in Fig. 4b, the two antenna sections formed by conductor portions 412, 414, 416 and 413, 415, 417, respectively, are symmetric, so that, for instance, both ends of the dipole antenna 41 are folded, the length of the straight portion 417 is equal to the length 419 of the straight conductor portion 416, et cetera. It is also possible, however, that the two antenna sections of a dipole antenna which is folded are not symmetric, so that, for instance, only one end of the respective dipole antenna is folded and/or the dimensions of the two antenna sections differ from each other.

Fig. 4c schematically and exemplarily shows a dipole antenna 42 with folded ends. In this case, two antenna sections of the dipole antenna 42 are connected via the excitation source 421. In this embodiment, the antenna sections comprise several conductive elements in the form of elongated conductors. For example, the antenna section on the right hand side of Fig. 4c comprises conductive elements 422, 422', 424 and 424'. These four conductive elements 422, 422', 424, 424' are interrupted by gaps, wherein in each of these gaps, an electrical component 426, 426', 426" is placed. The electrical components 426, 426', 426" can, for example, be any of the following: a resistor, a capacitor, an inductor, a lead or a combination of the aforementioned. The conductive elements 422, 422', 424, 424' are each formed by one straight elongated conductor in this example. The conductive elements 424, 424' and the electrical components 426', 426" can be regarded as the folded end of the respective antenna section. In the exemplary embodiment shown here, the right antenna section comprises straight conductive elements 422, 422', 424, 424', wherein the bent portion of the antenna section is formed by the electrical component 426'. The antenna sections of the dipole antenna 42 as shown in Fig. 4c are symmetric, so that, in particular, the antenna section on the left hand side of Fig. 4c also comprises straight conductive elements 423, 423', 425, 425' and electrical components 427, 427', 427" which are placed in the gaps between the conductive elements 423, 423', 425, 425', respectively. The electrical components 427, 427', 427" can, for example, be any of the following: a resistor, a capacitor, an inductor, a lead or a combination of the aforementioned.

The use of electrical components, such as the electrical components 426, 426', 426", 427, 427', 427" allows for adjusting the electrical properties of the dipole antenna 42 such as, for instance, the operating frequency, the electromagnetic phase profile and/or the load stability of the dipole antenna 42. In an example, the electrical components 426, 426", 427, 427" are capacitors, while the electrical components 426', 427' are inductors. In this example, the electrical properties of the dipole antenna 42 can be adjusted by adjusting the respective capacitance and inductance values, thereby improving the transmission and reception properties of the RF antenna for the respective use case. In particular, the respective capacitance and inductance values can be selected so that the dipole antenna 42 is improved, in particular optimized, for the respective use case. Examples of how to improve the electrical properties of a dipole antenna by using electrical components placed in gaps are provided below.

It shall be understood that, while the elongated conductors forming the conductive elements of the dipole antennas 40, 41 and 42 are shown schematically as thin wires, the actual cross-sectional profile of the elongated conductors can have different shapes. For instance, the cross-sectional profiles of the elongated conductors of the dipole antennas 40, 41 and 42 can be round, elliptical, rectangular or square. Furthermore, the cross-sectional profile of the elongated conductors can be full or hollow. For instance, the elongated conductors can comprise portions which are full, like a solid wire with round cross-sectional profile, and/or portions which are hollow, like a cylindrical wire. Fig. 4d schematically and exemplarily shows an embodiment of a dipole antenna 43 with folded ends in which the antenna sections each comprise a conductive element in the form of an elongated conductor with a rectangular cross-sectional profile. In particular, the elongated conductor is wide here, i.e. one of the dimensions perpendicular to the elongated dimension is much wider than the other dimension perpendicular to the elongated dimension. The wide dimension could thus be referred to as width 438 of the elongated conductor. In this embodiment, the electrical conductor could thus also be referred to as an electrically conductive ribbon or strip. The elongated conductors of the dipole antenna 43 each comprise straight conductor portions 432, 436, 433, 437 and bent conductor portions 434, 435 and the two antenna sections are connected via the excitation source 431. Increasing the width of the elongated conductor can increase the bandwidth of the dipole antenna 43.

The dipole antennas 41, 42, 43 are preferably folded on a dielectric substrate, which can provide a defined dielectric surrounding for the RF antennas and can, furthermore, provide mechanical strength and isolate conducting portions of the RF antennas which are folded.

The bent portions of the dipole antennas 41, 42 and 43 could also be described as defining a substantially 180-degree bending. However, it is also possible that at least one of the first RF antenna 4 and the second RF antenna 5 are dipole antennas which are folded, wherein the respective RF antenna comprises bent portions that could also be described as defining a substantially 90-degree bending. For instance, an antenna section of the respective RF antenna could comprise a conductive element in the form of an elongated conductor with two straight conductor portions, which are connected to each other via a bent conductor portion, wherein the bent conductor portion describes a 90-degree bending, so that the first straight conductor portion and the second straight conductor portion are arranged perpendicular to each other. Furthermore, the dipole antennas 41, 42 and 43 as shown schematically in Figs. 4b, 4c and 4d seem to have certain relative dimensions. It is also possible that the actual relative dimensions are different, however. For instance, the distance between the straight conductor portions 412 and 416 or the distance between the straight conductor portions 432 and 436 could be much larger than what seems to be indicated in Figs. 4b and 4d. Also the distance between the conductive elements 422 and 424' could be much larger than what seems to be indicated in Fig. 4c.

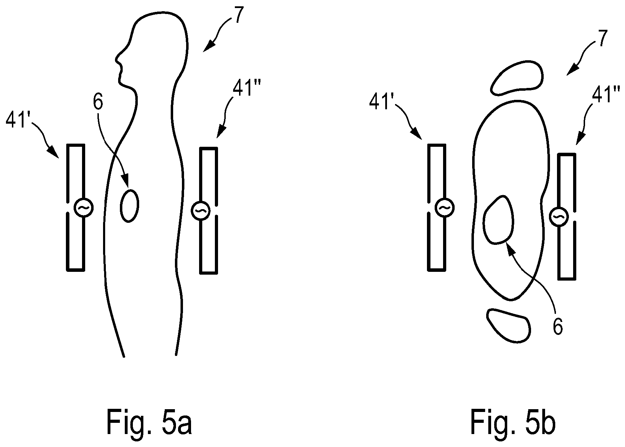

When one of the dipole antennas described with reference to Figs. 4b, 4c, 4d is used as the first RF antenna and/or the second RF antenna, the ends are preferably folded such that in use, when the subject 7 is located in between the opposing RF antennas, the ends are folded away from the subject 7. Figs. 5a and 5b show schematically and exemplarily the subject 7 positioned in between two dipole antennas 41', 41" with folded ends, wherein the ends are folded away from the subject. In an embodiment, the dipole antenna 41' could be the first RF antenna of the system 1 for determining a physiological parameter, while the dipole antenna 41" could be the second RF antenna of the system 1 for determining a physiological parameter. As shown in Figs. 5a and 5b, the dipole antennas 41', 41" are preferably aligned substantially parallel to each other. The system 1 can be configured such that the two RF antennas are arranged vertically, as shown in the sagittal plane view of Fig. 5a, or the system 1 can be configured such that the two RF antennas are arranged horizontally, as shown in the transverse plane view of Fig. 5b. Preferably, the first and second RF antennas of the system 1 are arranged horizontally. For the sake of clarity, Figs. 5a and 5b do not show other elements of the system 1 such as the RF instrument 2 of the measurement device 8 or the determination device 12.

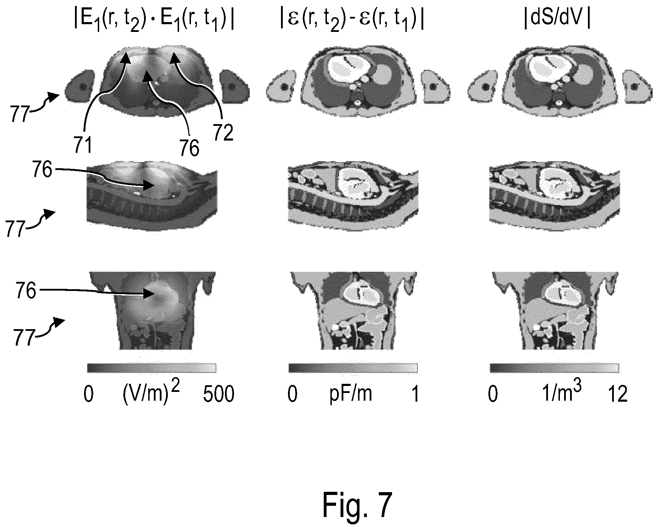

In the following examples, the performance of several RF antenna designs for a system for determining a physiological parameter will be described with reference to Figs. 7 to 14. Special emphasis will be given to the performance in terms of coverage of the region of interest, the stability with respect to load variations and cancellation of the electromagnetic phase profiles. Furthermore, a method for improving dipole antennas with folded ends as well as experimental results obtained with dipole antennas with folded ends will be described.

In the following examples, the aim can be to determine a physiological parameter, in particular quantify a hemodynamic parameter such as stroke volume, by using systems for determining a physiological parameter of a subject based on RF measurements, for instance embodiments of the system 1. The corresponding measurement method could also be referred to as RF sensing (RFS). RFS can be used, for example, to detect mechanical motion of the heart, because the changes in volume and position of the heart within a full cardiac cycle can result in changes of the impedance, reflection coefficients and coupling coefficients of one or more antennas placed close to the chest of a subject.

A theoretical description can be derived from the reaction theorem, resulting in the following equation: ${S}_{ij , t} - {S}_{ij , 0} = Δ {S}_{ij , t} = \frac{jω}{2 {a}_{i} {a}_{j}^{∗}} {\int }_{V} \left({ϵ}_{t , r}-{ϵ}_{0 , r}\right) {E}_{0 , r , i} ⋅ {E}_{t , r , j} dV$

In equation 2, Δ*Sᵢⱼ*(*t*) is the change in Sᵢⱼ (scattering coefficient) at timepoint t compared to reference timepoint 0. Note that by setting *i = j*, the reflection coefficient *S*₁₁ is evaluated. The normalization term includes the operating frequency *ω* and *a* is the forward power transmitted toward the antenna. The terms *ε* and *E* represent the spatiotemporal distributions of the complex tissue permittivity and electric field transmitted by the antenna. Changes in the complex spatial permittivity term (*ε_{t,r}* - *ε*_{0,*r*}) of the equation can be associated mainly with the heart movements and changes in the spatial electric field term (*E*_{0,*r,i*} · *E_{t,r,j}*) can be affected by the antenna design which can be manipulated to modify the sensitivity of the method.

Previous work described in "Measuring stroke volume with wearable RF antennas: a validation study with EM simulations and MRI." by B. R. Steensma et al., 31st Conference of the International Society of Magnetic Resonance in Medicine (2022), which is herewith incorporated by reference, showed that a loop antenna operating in the high field frequency range up to 300 MHz was sensitive to changes in stroke volume. However, loop antennas can have an electric field minimum in their center where the heart is usually located. Fig. 7 exemplarily illustrates the electric field term (first column), permittivity term (second column), and differential reflection coefficient (third column) of equation (1) for the situation of a single loop antenna placed on the front side of the thorax of a subject 77, wherein the loop antenna transmits an RF signal. In Fig. 7, *t₁* and *t₂* denote the diastolic and systolic parts of the heart cycle, respectively, and the first row shows the transverse plane, the second row shows the sagittal plane and the third row shows the coronal plane through the thorax of the subject 77. The electric field maxima, as seen for instance as bright regions 71, 72 in the transverse plane depiction in the upper left panel of Fig. 7, do not occur at the position of the heart 76.

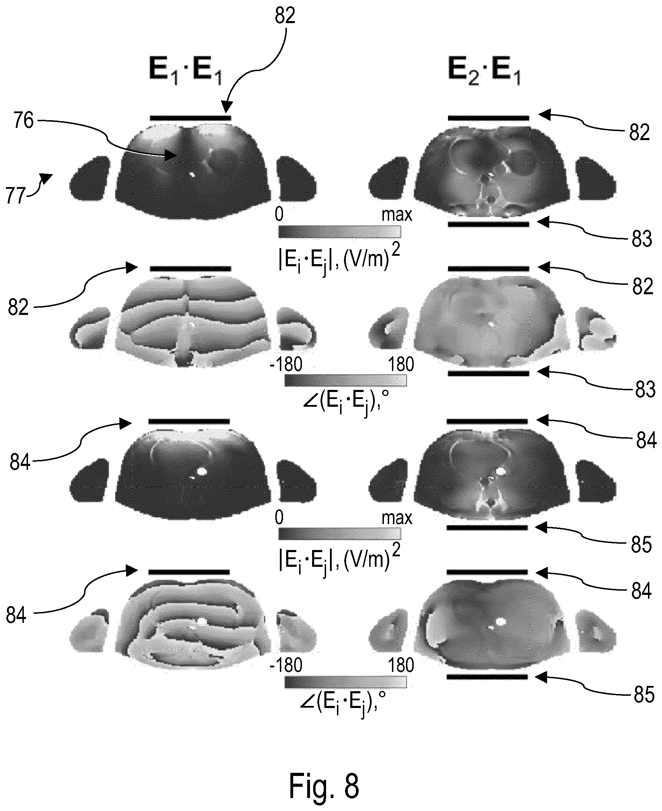

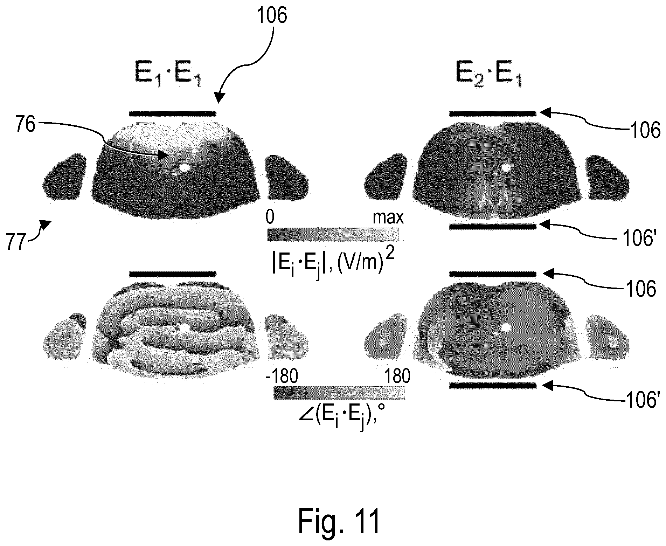

A dipole antenna can be configured such that its nearfield radiation pattern provides a more uniform and more complete coverage of the heart. Fig. 8 exemplarily shows the simulated spatial sensitivity of RFS on a human model of a subject 77 using RF radiation at 433 MHz. The simulations shown in Fig. 8, as well as similar simulations shown in Figs. 7 and 11, have been obtained by electromagnetic finite difference time dome (FDTD) simulations (Sim4Life, Zurich Medtech, Zurich, Switzerland) performed on the Duke voxel model as described, for instance, in the articles "The Virtual Family - development of surface-based anatomical models of two adults and two children for dosimetric simulations." by A. Christ et al., Physics in Medicine & Biology, Volume 55, Issue 2, Page N23 (2009), which is herewith incorporated by reference, and "4D XCAT phantom for multimodality imaging research." by W. P. Segars et al., Medical Physics, Volume 37, Issue 9, Pages 4902-4915 (2010), which is herewith incorporated by reference. The left column of Fig. 8 exemplarily shows results for the situation of a single RF antenna being placed on the front of the thorax of the subject 77, wherein in the first and second row the RF antenna 82 is a loop antenna and in the third and fourth row the RF antenna 84 is a dipole antenna. The right column of Fig. 8 exemplarily shows results for the situation of a first RF antenna being placed on the front of the thorax and a second RF antenna being placed on the back of the thorax of the subject 77, wherein in the first and second row the RF antennas 82 and 83 are loop antennas and in the third and fourth row the RF antennas 84 and 85 are dipole antennas. The RF antenna 84 could, for instance, be the first RF antenna of the system for determining a physiological parameter and the second RF antenna 85 could, for instance, be the second RF antenna of the system for determining a physiological parameter. The first and the third row of Fig. 8 show the amplitude of the electric field term, while the second and fourth row of Fig. 8 show the phase of the electric field term. With only one loop antenna 82, the electric field term has a signal void in the center of the heart, whereas the setup with only one dipole antenna 84 covers mainly the frontal part of the heart volume. Additionally, the use of only one antenna 82, 84 results in strong spatial variation of phase. In this example, the configuration with two dipole antennas 84, 85 (cf. the bottom two illustrations on the right-hand side of Fig. 8) is most beneficial in terms of amplitude and phase distribution homogeneity. In particular, the electromagnetic phase profiles of the two dipole antennas 84, 85 partly cancel out each other in this opposing configuration. In comparison, the electromagnetic phase profiles of the two loop antennas 82, 83 also cancel out each other, albeit to a lesser extent than the electromagnetic phase profiles of the dipole antennas (cf. the second illustration from the top on the right-hand side of Fig. 8). The spatial sensitivity of determining the physiological parameter, for instance, the stroke volume, can thus be more uniform over the structure when using two dipole antennas 84, 85 in an opposing configuration in this example. This allows for an improved determination of the physiological parameter.

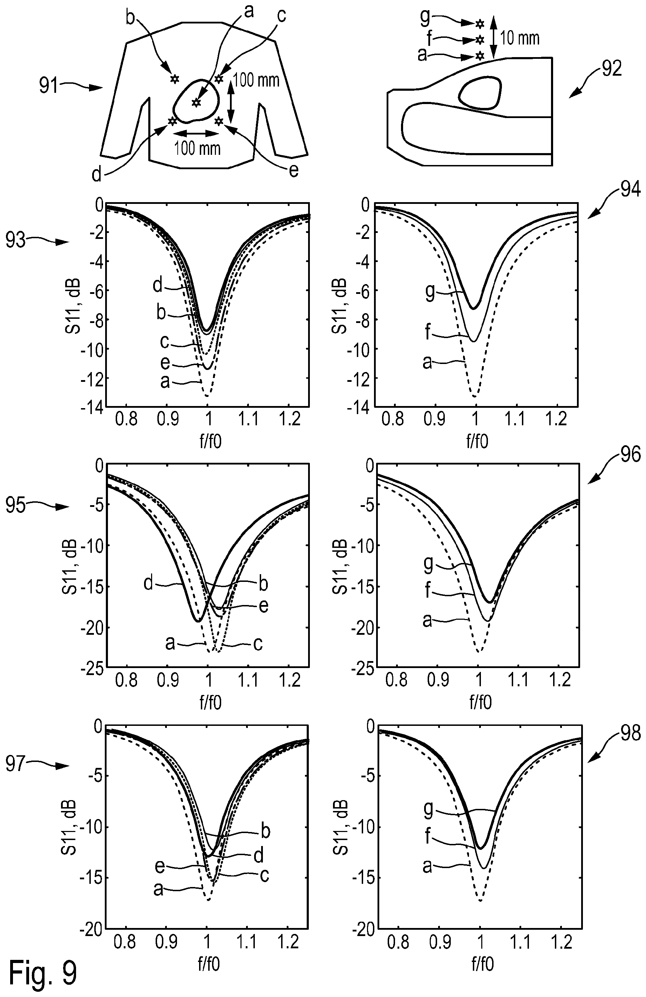

Another aspect that is preferably considered is the stability of the resonant frequency of the RF antennas, in particular with respect to load variations. Fig. 9 shows exemplary simulation results of the reflection coefficient *S₁₁* for different RF antenna configurations and variations in placement. In particular, by varying the placement with respect to the subject in the simulations, a variation in load can be simulated. Graphs 93 and 94 show simulation results for a loop antenna with 100 mm diameter tuned using capacitors. Graphs 95 and 96 show simulation results for a straight dipole antenna, i.e. a dipole antenna which is not folded, tuned to 433 MHz by adapting the geometrical dimensions accordingly. Graphs 97 and 98 show simulation results for a dipole antenna which is folded, wherein the ends of the dipole antenna are folded away from the subject, tuned to 433 MHz by adapting the geometrical dimensions. Each antenna was tuned when centered over the heart at a 5 mm distance from the Duke XCAT model with realistic heart motion as described in, for instance, the article "4D XCAT phantom for multimodality imaging research." by W. P. Segars et al., Medical Physics, Volume 37, Issue 9, Pages 4902-4915 (2010), which is herewith incorporated by reference. The position of a center point of the respective antenna for tuning is indicated in the schematic illustrations 91, 92 of the thorax by location a. After that, each antenna was shifted by 50 mm in the plane in left-right and/or up-down directions from the original location a, as indicated by locations b, c, d and e, or by 5 mm and 10 mm away from the model, as indicated by locations f and g. For each of the locations a to g, the reflection coefficient *S₁₁* was calculated as a function of the excitation frequency *f*, normalized by the resonant frequency *f₀* of 433 MHz. The individual curves for the reflection coefficient *S₁₁* are marked according to the location of the center point of the respective antenna. The results in graphs 93 and 94 show that the loop antenna is barely affected by the repositioning of the antenna, i.e. the load variation. Mainly the Q-factor changes, but the resonant frequency varies by less than 1%. The results in graphs 95 and 96 show that the resonant frequency of the straight dipole antenna changes up to 10%. The results in graphs 97 and 98 show that the dipole antenna with ends folded away from the subject has an improved stability with resonant frequency shifts of less than 5% between various positions.

The stability of an RF antenna, in particular a dipole antenna or a dipole antenna with folded ends, with respect to load variations can further be improved when the RF antenna comprises further electrical components, in particular, one or more capacitors and/or inductors. For instance, the antenna sections of a dipole antenna can comprise conductive elements, for instance, elongated conductors, and electrical components, in particular capacitors and/or inductors, can be placed in gaps between the conductive elements. Preferably, the electrical components placed in the gaps are selected so as to improve, in particular maximize or minimize, a parameter describing the stability and/or performance of the antenna. A corresponding method for improving the antenna can, for instance, aim at minimizing a shift of the resonant frequency and/or a reflection coefficient at a predetermined frequency under load variation. The method for improving the antenna can also take into account several parameters, for instance, by minimizing a weighted sum of parameters such as a shift of the resonant frequency and a reflection coefficient at a predetermined frequency under load variation. Furthermore, the method for improving the antenna can take into account a plurality of parameter values obtained with varying load. A plurality of parameter values can be taken into account, for instance, by minimizing an average of the plurality of parameter values, wherein an average can be, for instance, an arithmetic mean, a median or a generalized mean such as the quadratic mean of the plurality of parameter values. The plurality of parameter values can refer to, for instance, a plurality of shifts in the resonant frequency obtained by changing the antenna placement with respect to, for instance, a subject's body. The plurality of parameter values can also refer to, for instance, a plurality of reflection coefficients at a predetermined frequency obtained by changing the antenna placement with respect to, for instance, a subject's body.

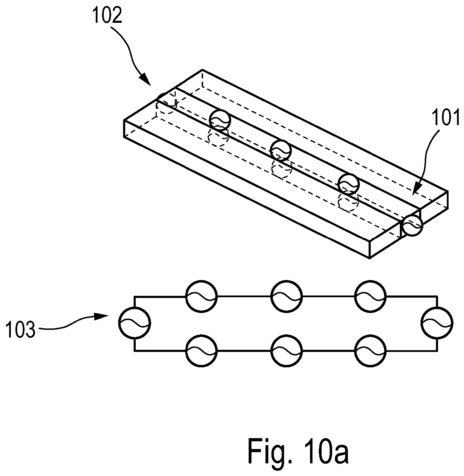

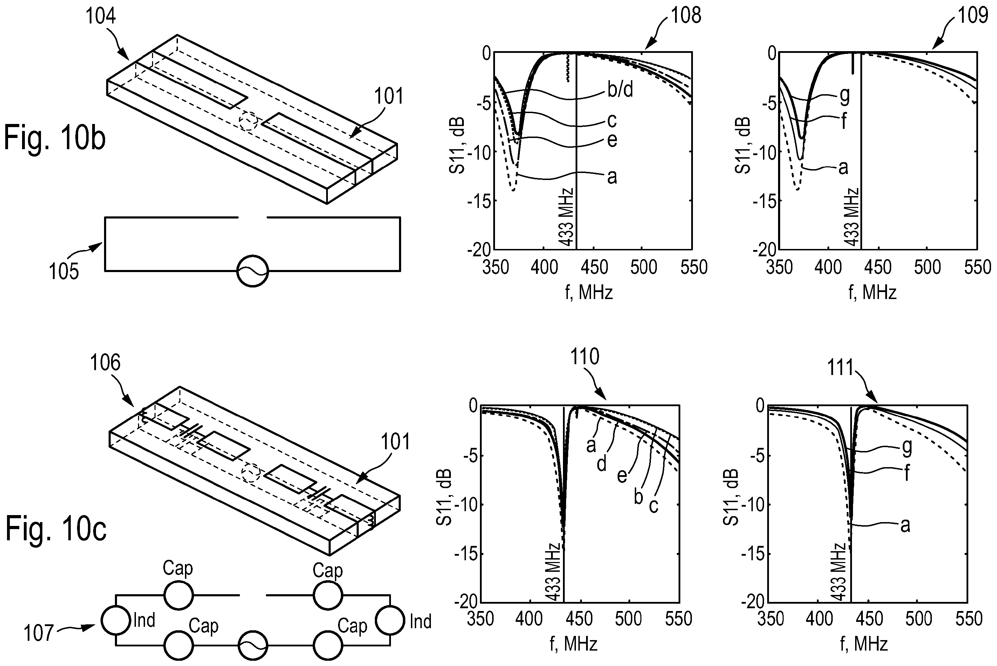

An exemplary method for improving a dipole antenna with folded ends will be explained in detail in the following. In this example, a dipole antenna 106 with the ends folded away from the subject was modelled with lumped elements (LEs) implemented into the antenna design. A schematic illustration of the dipole antenna 106 is shown in Fig. 10c, a dipole antenna 104 without lumped elements as a reference design is shown in Fig. 10b and a general design 102 used in the method for improving the dipole antenna is shown in Fig. 10a. A PMMA substrate, especially a Plexiglas substrate, 101 with a length of 180 mm, a width of 30 mm, a height of 4 mm and a relative permittivity of 3.4 was modeled as a housing or substrate on which the dipole antennas are folded. Circuit co-simulations were used to identify improved parameters for the lumped elements of the dipole antenna 106. Eight ports were used in the general design as shown in the simplified schematic 103 in Fig. 10a. For the dipole antenna 106, seven ports were replaced with lumped elements or short/open connections, and one port was used as a source in the co-simulation in postprocessing. In particular, as shown in the simplified schematic 107 in Fig. 10c, 2 ports were replaced by inductors (Ind), 4 ports were replaced by capacitors (Cap), one port was replaced by an open circuit and one port was used as a source to obtain the design of the dipole antenna 106.

For calculating the resonant frequencies and the reflection coefficients, the dipole antenna 106 was placed in the same locations a to g as described before with reference to Fig. 9. For all setups the S-matrix (8x8) was calculated. The CoSimPy Python library was used for co-simulation to replace ports with lumped elements or short/open connections as described in, for instance, the article "CoSimPy: An open-source python library for MRI radiofrequency Coil EM/Circuit Cosimulation." by U. Zanovello et al., Computer Methods and Programs in Biomedicine, Volume 216, Page 106684 (2022), which is herewith incorporated by reference. The following function was used as a goal function: ${f}_{goal} \left(LEs\right) = α {\left‖1-\frac{{f}_{res}^{i} \left(LEs\right)}{{f}_{0}}\right‖}_{2} + β {\left‖{S}_{11 , {f}_{0}}^{i}\left(LEs\right)\right‖}_{2}$

In equation 2, *α* and *β* correspond to the weights of the frequency shift term ${\left‖1-\frac{{f}_{res}^{i} \left(LEs\right)}{{f}_{0}}\right‖}_{2}$ and matching term ${\left‖{S}_{11 , {f}_{0}}^{i}\left(LEs\right)\right‖}_{2}$, respectively, ${f}_{res}^{i} \left(LEs\right)$ is the resonant frequency of the antenna, *f*₀ = 433 MHz, ${S}_{11 , {f}_{0}}^{i} \left(LEs\right)$ is the reflection coefficient at *f*₀ and *i* = 1 ... 7 are the different positions of the antenna as described and shown before in Fig. 9. A multi-variable scalar optimization function ("scipy.optimize.minimize") with a Constrained Optimization BY Linear Approximation ("COBYLA") method was used to minimize the objective function. Furthermore, to obtain the reference design of the dipole antenna 104 as shown in the simplified schematic 105 in Fig. 10b, 6 ports were replaced by short connections, one port was replaced by an open circuit and one port was used as a source.

Graphs 108 and 109 in Fig. 10b show the results of the stability test before improving the dipole antenna design, i.e. the results obtained with the reference dipole antenna 104. Not taking into account the resonant frequency of the dipole antenna 104, up to 15 MHz resonant frequency shifts are obtained when, starting from location a, in-plane shifts to locations b, c, d and e and out-of-plane shifts to locations f and g were performed. For improving the dipole antenna 106 the positions and values of the lumped elements were varied to achieve stability. The dipole antenna 106 with minimal frequency shift comprises four capacitors of 6 pF each and two inductors of 10 nH each, which were implemented in the dipole antenna design. This resulted in a frequency shift of less than 1 MHz for all antenna positions as shown in graphs 110 and 111 in Fig. 10c. After optimization, an additional electromagnetic FDTD simulation was performed with the dipole antenna 106 to verify the improved electric field distribution and phase distribution. The results of the FDTD simulation are shown in Fig. 11, wherein the results in the left-hand column were obtained with only one dipole antenna 106 placed on the front of the thorax of the subject 77 and the results in the right-hand column were obtained with one dipole antenna 106 placed on the front of the thorax and one identical dipole antenna 106' placed on the back of the thorax of the subject 77. The electric field term amplitude obtained with the improved dipole antenna 106 with folded ends are shown in the upper row and the corresponding phase distributions are shown in the bottom row of Fig. 11. In particular, the configuration with two opposing dipole antennas 106, 106' provides for a very homogeneous amplitude and phase distribution and the electromagnetic phase profiles of the two dipole antennas 106, 106' cancel out each other to a very high degree.

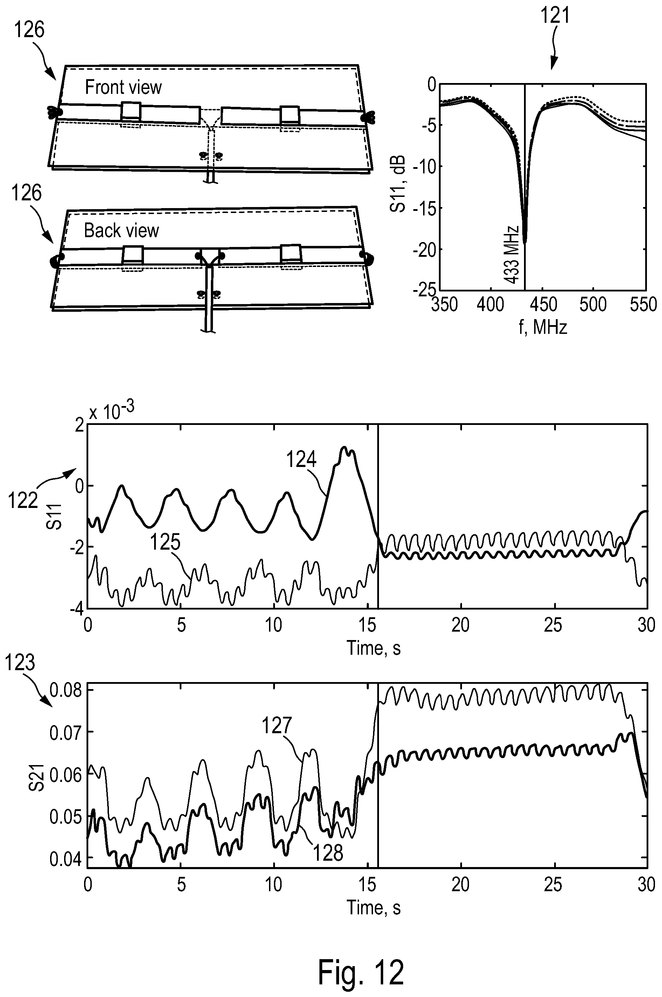

A dipole antenna 126 with folded ends, which was designed to realize the features of the improved dipole antenna 106, was built for experimental measurements. Fig. 12 illustrates the dipole antenna 126 from the front and the back. The dipole antenna 126 is folded on a dielectric substrate with a length of 180 mm, a width of 30 mm and a height of 4 mm, with a relative permittivity of 3.4. The dielectric substrate of the dipole antenna 126 is made from poly(methyl methacrylate) (PMMA), especially Plexiglas. However, for a realization of the improved dipole antenna 106, also a flexible substrate could be used such as, for instance, a substrate made from silicone. The dipole antenna 126 has an overall length of 180 mm. A gap between the two folded ends of the dipole antenna 126 is about 5 mm in width. Capacitors with values of 6 pF and hand-wound inductors with an estimated inductance of 10 nH were used in the dipole antenna 126, based on the simulation results. To experimentally check the stability with respect to load variations, the dipole antenna 126 was centered over the heart of a volunteering subject and then moved in the plane as it was done virtually for the numerical simulations. The reflection coefficient was measured for each placement using a tabletop vector network analyzer (Planar TR1300/1, Copper Mountain Technologies, IN, US). In terms of stability, the dipole antenna 126 showed the results that were expected based on the numerical simulations. Experimental measurements of the reflection coefficient *S₁₁* as a function of frequency *f* are shown in graph 121 in Fig. 12. In particular, when the dipole antenna 126 was moved in-plane and close to the volunteer, the changes in resonant frequency were less than 1 MHz. A setup with two dipole antennas identical to dipole antenna 126 was also tested on a volunteering subject, wherein the thorax of the subject was placed in between the two dipole antennas. The recorded signal graph 122 shows the real part 124 and the imaginary part 125 of the reflection coefficient *S₁₁* over time. The recorded signal graph 123 shows the real part 128 and the imaginary part 127 of the coupling coefficient *S₂₁* over time. This measurement could also be referred to as RFS measurement. Up to a time of approximately 16 s, the subject was breathing freely and the real and imaginary parts of *S₁₁* and *S₂₁* are affected by both respiratory and cardiac motion. At a time of approximately 16 s, the subject stopped breathing and during the following breath-hold the real and imaginary parts of *S₁₁* and *S₂₁* are solely associated with cardiac motion. As will be described in more detail below, the signals *S₁₁* and *S₂₁* could be used to determine a physiological parameter such as the stroke volume. In an example, the two dipole antennas with folded ends identical to dipole antenna 126 could form the RF module of the system for determining a physiological parameter and the tabletop vector network analyzer could form the corresponding RF instrument, wherein the signals *S₁₁* and *S₂₁* could be the provided motion signal related to a mechanical movement of a structure within the subject.

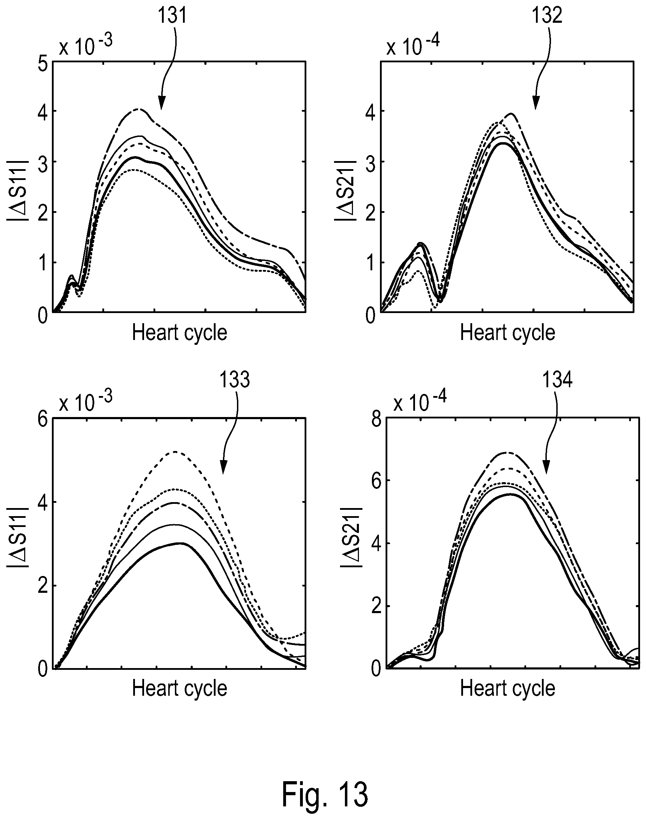

The setup with two dipole antennas was also used for a reproducibility test with two healthy subjects (23 years old, male, BMI 21 kg/m² and 33 years old, male BMI 20 kg/m²). RFS measurements of *S₁₁* and *S₂₁* were performed 5 times after repositioning the dipole antennas at the same place on the chest. One of the antennas was placed in front of the chest centered over the heart, and the other one was placed in the back also centered over the heart. Both dipole antennas were fixed with a neoprene belt to ensure fixation to the chest. RFS and ECG measurements were collected simultaneously. The RFS signal was averaged over 5 heart cycles during an end-expiration breath hold for each measurement. The mean and standard deviation values of the amplitude of *ΔS₁₁* and *ΔS₂₁* were evaluated. Fig. 13 shows the results of these measurements, wherein graphs 131 and 132 show the recorded signals for volunteer 1 and graphs 133 and 134 show the recorded signals for volunteer 2. The results of the reproducibility test show some variation in the amplitude of the RFS signal, wherein the variation is more prominent in the *ΔS₁₁* signal compared to the *ΔS₂₁* signal. The coefficient of variation was evaluated as the ratio of the standard deviation and mean value of the RFS amplitude for all five repetitions. The coefficient of variation for *ΔS₂₁* was lower for both volunteers (6.4 % for volunteer 1, 8.7 % for volunteer 2) than the coefficient of variation for *ΔS₁₁* (13.5 % for volunteer 1, 20.3 % for volunteer 2), which can be attributed to the more homogeneous amplitude and phase distribution of the electric field in the case of the setup with two dipoles.

The simulation results and experimental results presented with reference to Figs. 7 to 13 show that an RF module comprising two RF antennas in an opposing configuration can be configured to have a particularly high and uniform spatial sensitivity for a mechanical movement of a structure within a subject. Moreover, these results indicate that an RF module comprising two RF antennas which are dipole antennas with folded ends can be configured to have an even higher and more uniform spatial sensitivity. The dipole antennas with folded ends preferentially comprise electrical components, in particular a capacitor and/or an inductor, which are connected in series by using conductors. As explicitly demonstrated for exemplary embodiments of dipole antennas, such electrical components allow for adjusting the electrical properties of the dipole antennas, thereby improving the transmission and reception properties of the RF antenna for the respective use case.

In an embodiment of the system 1 for determining a physiological parameter of the subject 7, the first RF antenna 4 and the second RF antenna 5 of the RF module 3 are dipole antennas with folded ends, in particular dipole antennas with folded ends identical to the dipole antenna 126. In this embodiment, the physiological parameter can be the stroke volume and the first and second RF antennas can be configured so that the thorax of the subject 7 is located in between the opposing RF antennas 4, 5, i.e. similar to the configuration described with reference to Figs. 12 to 14. In this embodiment, the RF instrument 2 can be a vector network analyzer configured to provide as a motion signal a complex signal like a complex reflection coefficient signal or complex coupling coefficient signal. In particular, the vector network analyzer can be configured to provide as a motion signal two complex signals like the complex reflection coefficient signal *S₁₁* and the complex coupling coefficient signal *S₂₁* exemplarily shown in Fig. 12 as curves 124, 125, 127 and 128, respectively.

In this embodiment, the processor 15 of the determination device 12 is configured to identify, for each complex signal, a first subsignal of the respective complex signal having a distinct phase shift (for example 90 degrees) with respect to a second subsignal of the respective complex signal and to determine the physiological parameter based on the separated subsignals, which can also be referred to as processed motion signal. In this embodiment the physiological parameter is the stroke volume, wherein for this reason it is desired to have a subsignal which is related to the mechanical movement of the heart 6 within the subject 7, wherein an influence by other movements within the subject 7 should be as small as possible. It has been found that in the respective complex signal the contribution caused by cardiac motion has a distinct phase shift with respect to a contribution caused by respiratory motion. Thus, by identifying the phase shift of the first subsignal with respect to a second subsignal of the respective complex signal, the influence of respiratory motion on the signal, which is finally used for determining the stroke volume, can be strongly reduced or even eliminated.

The processor 15 can be further configured to apply a blind source separation technique to separate the subsignals out of the multiple complex signals received by the antenna module 3, for example by applying ICA or SOBI. Furthermore, the processor 15 can be configured to apply a frequency filtering to the first subsignal like a band-pass filtering, a low-pass filtering, a high-pass filtering or Kalman filtering, in order to further reduce contributions to the first subsignal, which are not caused by the mechanical movement of the heart 6.

The resulting two first subsignals, which are obtained based on the two complex signals measured by using the two RF antennas 4, 5, can be combined to a combined signal by, for example, blind source separation like PCA. In another embodiment, the first subsignal with the least contribution from the second subsignal is selected based on spectral analysis. In particular, the processor can be configured to determine which first subsignal, i.e. which processed motion signal, has the largest deviation between a value in an expected frequency range, in which the respective motion is expected, and values outside of this frequency range. For instance, the processor can apply a Fourier transform for carrying out this comparison. The comparison can be between the expected frequency range and all values outside of the frequency range or the comparison can be between the expected frequency range and another unwanted frequency range in which an unwanted motion contribution is expected. For instance, if it should be determined which first subsignal, i.e. which processed motion signal, is related to cardiac motion, the expected frequency range can be 0.7 Hz to 1.5 Hz and the further unwanted frequency range with the unwanted motion being, for instance, respiratory motion can be 0.15 Hz to 0.25 Hz. The other way around, if it should be determined which motion signal is caused by respiratory motion, the expected frequency range being, in this case, for instance 0.15 Hz to 0.25 Hz can be compared with values in the in this case unwanted cardiac frequency range from, for instance, 0.7 Hz to 1.5 Hz or with all values outside of the expected frequency range. Thus, for instance, a peak value or average value in the expected frequency range can be compared with the average value or peak value in the unwanted frequency range or of all frequencies outside of the expected frequency range. The comparison can be carried out by division or subtraction. The first subsignal, for which the comparison provided the largest deviation to the values in the expected cardiac frequency range, is determined as being the processed motion signal that is related to mechanical movement of the heart. Correspondingly, the first subsignal, for which the comparison yields the largest deviation with respect to the respiration frequency range, is regarded as being the motion signal that is related to mechanical motion of the lungs. In other words, the first subsignal, for which the comparison resulted in a higher value in the expected cardiac frequency range, is regarded as being the cardiac processed motion signal and the first subsignal, for which the comparison resulted in a higher value in the expected respiration frequency range, is regarded as being the respiration processed motion signal.

In this embodiment the model providing module 14 is configured to provide a linear model as the model. Thus, a linear function is provided, which relates the processed motion signal to the stroke volume. Correspondingly, the processor is configured to determine the stroke volume based on the provided linear model and the processed motion signal, wherein the provided model has been determined before by training which also could be named calibration. An embodiment of a training system for training the model to be provided by the model providing module will be described exemplarily in the following.

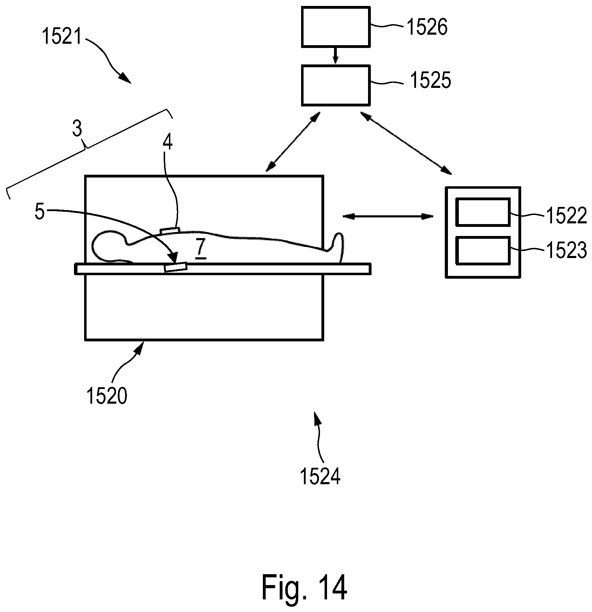

Fig. 14 shows schematically and exemplarily a training system 1521 for training the model to be provided by the model providing module 14. The training system 1521 comprises a training physiological parameter measurement device 1524 for measuring a training physiological parameter of the subject 7 being, in this embodiment, the stroke volume.

The training physiological parameter measurement device 1524 comprises a magnetic resonance (MR) signals generation device 1520 which preferably uses the RF antenna module 3, i.e. the first RF antenna 4 and the second RF antenna 5, of the measurement device 8 for determining the stroke volume. The training physiological parameter measurement device 1524 further comprises a controller 1522 for controlling the MR signals generation device 1520 and a training physiological parameter determination module 1523 for determining the training physiological parameter based on the generated MR signals. In this embodiment the training physiological parameter determination module 1523 is configured to determine the stroke volume based on the MR signals generated by the MR signals generation device 1520. For determining the stroke volume, the MR signals generation device 1520, the controller 1522 and the training physiological parameter determination module 1523 can be configured to reconstruct MR images based on the MR signals and to determine the stroke volume based on the reconstructed MR images. In an embodiment, for determining the stroke volume, the MR signals generation device 1520, the controller 1522 and the training physiological parameter determination module 1523 are configured to operate in accordance with known techniques like the technique disclosed in the article by Groepenhof et al., Physiological Measurements, 2007, 28(1):1-11 or in the article by Dornier et al., European Radiology, 2004 14(8):1348-52, which are herewith incorporated by reference. The MR signals generation device 1520 can be a device of a standard MR system.

In standard MR systems RF antennas are used to excite nuclear spins and detect signals emitted back by the magnetized spins. Since the RF antennas of MR systems are also sensitive to physiological motion, they can be used to detect and correct for physiological motion in magnetic resonance imaging (MRI). This is described, for instance, in the article "The rf coil as a sensitive motion detector for magnetic resonance imaging" by D. Buikman et al., Magnetic Resonance Imaging, Volume 3, Pages 281 to 289 (1988) which is herewith incorporated by reference. In an embodiment, the MR signals generation device 1520 operates at the same frequency as the RF antennas 4, 5 of the RF antenna module 3, so that the same RF antennas 4, 5 can be used for determining, for instance, the stroke volume by using MRI and measuring a motion signal by using the measurement device 8. Quantitative parameters like the stroke volume of the heart can thus be measured simultaneously with MRI and the measurement device 8. It is also possible, however, that the MR signals generation device 1520 operates at a different, in particular lower, frequency than the RF antennas 4, 5 of the RF antenna module. In this case, the training physiological parameter measurement device 1524 preferentially comprises RF antennas different from the RF antennas 4, 5, wherein these RF antennas are configured for determining the training physiological parameter, for instance, the stroke volume, by using MRI. It is still possible that the training physiological parameter is measured using MRI and the motion signal is measured using the measurement device 8 simultaneously. It is also possible that the training physiological parameter and the motion signal are measured one after another. For instance, in a first measurement a motion signal can be measured using the measurement device 8 and in a subsequent second measurement the training physiological parameter can be measured using MRI.

In another embodiment, also another training physiological parameter measurement device can be used. For example, the training physiological parameter measurement device can also be a Doppler echocardiographic device like the echocardiographic device disclosed in the article "Comparative accuracy of Doppler echocardiographic methods for clinical stroke volume determination" by Jonathan Dubin et al., American Heart Journal, Volume 120, Issue 1, Pages 116 to 123 (1990), which is herewith incorporated by reference. In this case, the stroke volume is considered as the training physiological parameter. The training physiological parameter measurement device can also be a Fick device, a dye dilution device or a thermodilution device as described in the article "Thermodilution Cardiac Output: A 15 Concept over 250 Years in Making" by E. Argueta et al., Cardiology in Review, Volume 27, Issue 3, Pages 138 to 144 (2019), which is also herewith incorporated by reference. For this example, cardiac output is used as a training physiological parameter.

The training system 1521 further comprises a model providing module 1526 configured to provide an adaptable model to be trained, wherein the model provides, as an output, a physiological parameter, if, as an input, a motion signal is provided. In this embodiment the model is a linear model of the type SV=ax+b, where x is, for example, the amplitude of the motion signal, the amplitude of the derivative of the motion signal, the area under a curve of the motion signal, the root-mean-square value or another quantity derived from the motion signal. In particular, x can be a quantity derived from the motion signal, wherein the motion signal is the processed motion signal. SV is the stroke volume, which preferentially is defined as the volume of blood pumped per beat from the left ventricle, and a, b are adaptable parameters which are adapted during the training process. For instance, the processed motion signal is the one where the first subsignal is present most strongly in the frequency domain, i.e. has the largest amplitude, in other words, in an example, it is the above mentioned cardiac processed motion signal.

Having the motion signal as input of the model preferentially means that one or more characteristics of the motion signal are input into the model like the amplitude of the motion signal, the amplitude of the derivative of the motion signal, the area under a curve of the motion signal, the root-mean-square value or another quantity derived from the motion signal, as mentioned above. Moreover, the motion signal can have been processed or not.

For determining the parameter "area under a curve of the motion signal", the processor can be configured to detect peaks of the processed motion signal, in order to identify individual periods of the oscillating processed motion signal. For detecting the peaks known peak detection algorithms can be used like the algorithm disclosed in the article "A semi-automatic method for peak and valley detection in free-breathing respiratory waveforms" by W. Lu et al., Medical Physics, Volume 33, Issue 10, Pages 3634-3636 (2010), which is herewith incorporated by reference. The processor can be further configured to, for each peak-to-peak interval, integrate the total amplitude over time, in order to thereby determine the area under the curve. Thus, the respective part of the processed motion signal between two neighboring peaks is regarded as being the "curve", wherein the integral value obtained by integrating the total amplitude over time between the two neighboring peaks is regarded as being the area under the respective curve. The total amplitude is defined as the difference between the maximum value and the minimum value of the respective curve.

In another embodiment, the model providing module can also be configured to provide another model like a Gaussian process regression model. As the Gaussian process regression model, a model as disclosed in the article "Gaussian processes for real-time 3D motion and uncertainty estimation during MR-guided radiotherapy" by N. Huttinga et al., Medical Image Analysis, ArXiv:2204.09873 (2022) can be used. Also in this case, the model is used for mapping the processed signal to the physiological parameter being, for instance, the stroke volume or another physiological parameter like the ventricular movement speed.

In order to train the respective model like the Gaussian process regression model, in a training or calibration phase a reference physiological parameter, i.e. the gold standard, is compared with a physiological parameter determined by using the model to be trained. If the model is a Gaussian process regression model, a distribution of functions, which is associated with a mean and a covariance matrix, are modified, until the physiological parameter obtained by using the modified Gaussian process regression model corresponds as good as possible to the reference physiological parameter. For more details regarding the modification of the Gaussian process regression model, reference is made to the above-mentioned article by N. Huttinga et al.

Furthermore, the training system 1521 comprises the measurement device 8 configured so that the subject 7 is located in between the opposing RF antennas of the RF antenna module 3 of the measurement device 8. For clarity reasons in Fig. 14 only the RF antenna module 3 of the measurement device 8 is shown. As mentioned above, the training physiological parameter measurement device 1524 and the measurement device 8 can be configured to use the same RF antenna module 3.

The training system 1521 further comprises a training module 1525 configured to a) determine a physiological parameter of the subject 7 based on the model to be trained and a motion signal provided by the RF instrument 2 and the RF antenna module 3 and b) modify the model such that a deviation between this determined physiological parameter and the training physiological parameter is reduced, in particular, minimized. Preferentially, the training module 1525 is configured to use the same processing of the signals, which is also applied by the processor 15 before the processor 15 uses the model as described above for determining the physiological parameter during an actual determination, i.e. after the training phase has been completed.

In this embodiment the training model is configured to determine the stroke volume of the subject 7 based on the model to be trained, which is preferentially a linear model, and a processed signal which has been determined as described above and to modify the model such that a deviation between this determined stroke volume and a stroke volume determined by using the MR signals generation device 1520, the controller 1522 and the determination device 1523 is reduced, in particular minimized.

The trained model is then used by the above described system 1 for determining a physiological parameter like the stroke volume of a subject. The system can perform dynamic determinations or measurements of the physiological parameter, in particular, of the cardiac output. In an embodiment the first RF antenna 4, the second RF antenna 5 and the RF instrument 2 are integrated into a wearable holder, in particular the wearable holder 10. In an example, the system 1 can be used to monitor the pumping function of the heart at home in patients with heart failure.

Especially when the physiological parameter to be determined is related to the cardi-orespiratory system, the processor 15 is preferably configured to achieve a separation of cardiac and respiratory signals, i.e. into a first subsignal being heart-related and a second subsignal being lung-related. Particularly if the measurement device 8 is used for measuring a complex reflection coefficient and/or a complex coupling coefficient, the resulting cardiac and respiratory signals are periodic and have a distinct phase difference of, for example, 90 degrees. In a preferred embodiment, the processor 15 is configured to perform a phase rotation such that the cardiac signal, i.e. the first subsignal, appears on the real axis and the respiratory signal, i.e. the second subsignal, mostly on the imaginary axis. To be more generic, the processor 15 can be configured to perform a transformation, in particular a 2x2 matrix transformation, on the complex signals measured by the measurement device 8 to achieve this.

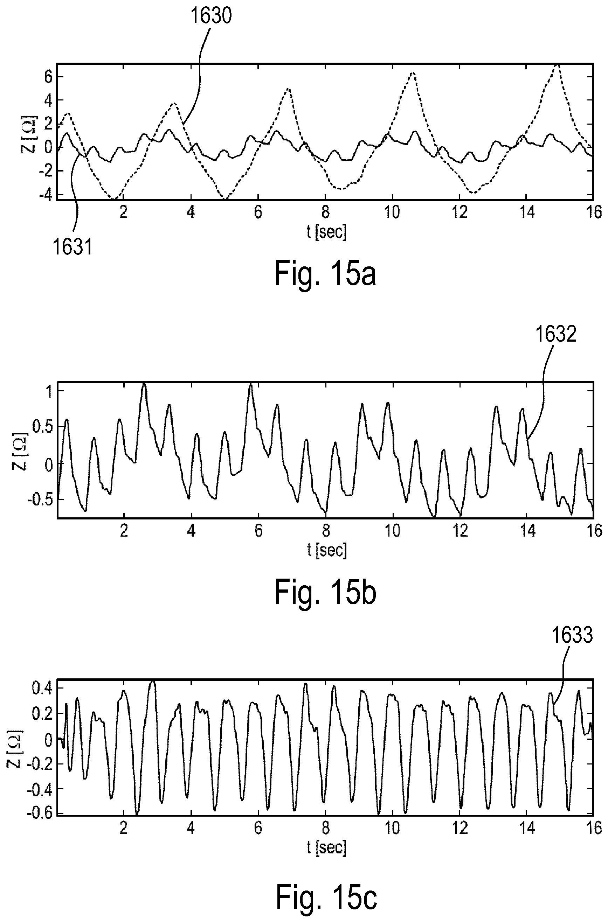

Fig. 15a schematically and exemplarily illustrates a measured complex signal Z over time, wherein in this figure the curve 1630 is the imaginary part of the signal Z and the curve 1631 is the real part of the signal Z. The complex signal Z can be, for instance, a complex coupling coefficient like the complex coupling coefficient *S₂₁* shown in graph 123 in Fig. 12. Fig. 15b illustrates the signal Z after the phase rotation has been carried out as described above, i.e. it shows the first subsignal along the real axis. The processor 15 can be further configured to exploit differences in spectral characteristics, in order to remove remaining contributions of motion that are not of interest. Thus, the processor 15 can be configured to perform filtering in the frequency domain. For instance, a bandpass filter, a low-pass filter, a high-pass filter or a Kalman filtering could be used. In an embodiment, a band-pass filter between 0.75 and 10 Hz is used, in order to filter out remaining components of a respiratory signal. This is illustrated in Fig. 15c. Thus, in Fig. 15c the curve 1633 results from filtering the curve 1632 shown in Fig. 15b by using a band-pass filter between 0.75 and 10 Hz.

A model can be used to predict stroke volume (SV, mL) from the measurement shown in Fig. 15c. For example, if the amplitude of the signal, the amplitude of the derivative of the signal or any other quantity derived from the signal in Fig. 15c is given as x, the stroke volume can be calibrated through a linear relationship as SV=a*x+b, where a and b are determined in a calibration phase. As described above, the stroke volume is measured with a reference instrument such as MRI or ultrasound in the calibration phase. This can be done under physiological stress, in order to increase the stroke volume during the measurements. At the same time, the parameter x is derived from the signal measured by using the first and second RF antennas 4, 5 and the RF instrument 2 which can be, for instance, a vector network analyzer. If SV and x are available for several different values of SV, the parameters a and b can be determined.

The above described system 1 can be used, for instance, to monitor the heart's pumping function in patients with heart failure at home. It can also be used to measure the heart rhythm and arrhythmia, or to quantify lung ventilation or edema, particularly to locally quantify lung ventilation or edema at home. For measuring the heart's pumping function, the stroke volume could be predicted based on a model of the effect of stroke volume on the measurements, for example the linear model SV=a*x+b. The same is possible for tidal volume, where the tidal volume (TV in mL) can be determined as TV = c*y+d, where y can be the amplitude of the respiration signal, and c and d are model parameters derived during a calibration measurement with a reference instrument such as spirometer. The TV and y can be measured during physiological stress, which will result in increasing TV over time. Based on this measurement, the parameters c and d can be determined. Parameters such as heart rate or respiratory rate can be derived from frequency domain analysis of the combined signals.

In another embodiment, the model providing module 14 is configured to provide another model which provides the relation between the motion signal and the physiological parameter. For example, the model providing the relation between the stroke volume SV and the amplitude of the RF signal or the model providing the relation between the tidal volume TV and the amplitude y of the RF signal, which is related to breathing, could be a Gaussian process regression model like the Gaussian process regression model described in the abovementioned article by Huttinga et al. The parameters of the Gaussian process regression model can be obtained in a training phase, wherein the parameters of the Gaussian process regression model are adapted such that the Gaussian process regression model outputs known given training physiological parameters, i.e. in this example known given SV or known given TV and optionally the uncertainty of the prediction, if, as an input, the amplitude of a respective RF signal is given.

In a further embodiment, the model providing module is configured to provide a model that provides, as an output, an echocardiography parameter if, as an input, the motion signal is provided. This model can be, for instance, a Gaussian process regression model. The echocardiography parameter is, for instance, the left ventricular outflow velocity. However, it can also be another echocardiography parameter. Echocardiography data being the left ventricular outflow velocity are described, for instance, in the article "Left ventricular outflow tract velocity time integral outperforms ejection fraction and Doppler-derived cardiac output for predicting outcomes in a select advanced heart failure cohort" by C. Tan et al., Journal of Cardiovascular Ultrasound, Volume 15, Issue 1, Page 18 (2017), which is herewith incorporated by reference. Also this model can be trained in a training phase, wherein the model is trained such that it outputs a known given echocardiography parameter like a known given left ventricular outflow velocity if, as an input, the motion signal is provided.

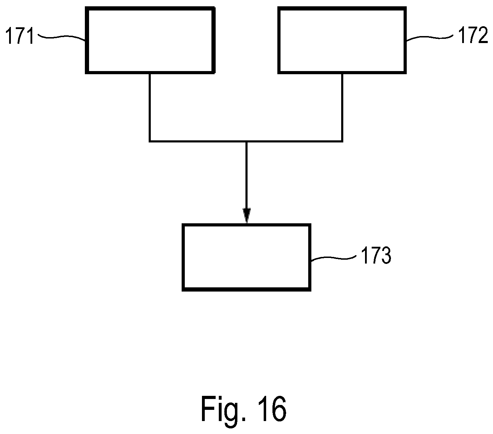

In the following, an embodiment of a method for determining a physiological parameter of a subject will be described with reference to a flowchart shown in Fig. 16. In step 171, a motion signal is provided, which is related to a mechanical movement of a structure like the heart 6 within the subject 7 by using the RF instrument 2, for instance, a vector network analyzer, and the RF antenna module 3 of the measurement device 8. In step 172, a model is provided, wherein the model has been trained to provide, as an output, a physiological parameter if, as an input, a motion signal is provided. The model is provided by the model providing module 14. In step 173, the physiological parameter is determined based on the provided model and the provided motion signal by the processor 15.

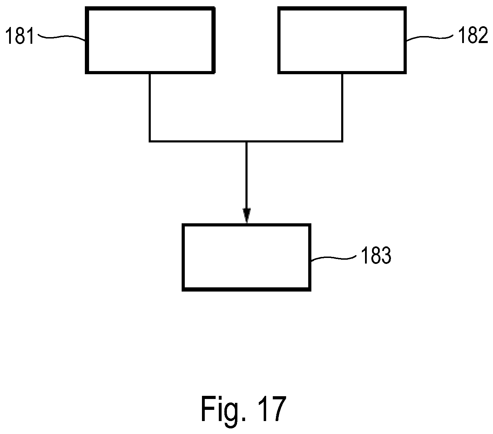

In the following, an embodiment of a training method for training a model to be used by the system for determining a physiological parameter will be exemplarily described with reference to a flowchart shown in Fig. 17. In step 181, a training physiological parameter of a subject is measured by the training physiological parameter measurement device 1524. For instance, by using MRI, a stroke volume of the heart is determined as the training physiological parameter. At the same time, a motion signal is provided, which is related to a mechanical movement of a structure within a subject, by using the measurement device 8. For instance, complex RF signals are measured, which are related to the mechanical movement of the heart. In step 182, a model to be trained is provided by a model providing module, wherein the model provides, as an output, a physiological parameter if, as an input, a motion signal is provided. In step 183, a physiological parameter of the subject is determined based on the model to be trained and the motion signal provided by the measurement device and the model is modified such that a deviation between the determined physiological parameter and the training physiological parameter is reduced, wherein this step is carried out by the training module 1525. For instance, the model can be adapted such that a deviation between a stroke volume measured by the training physiological parameter measurement device 1524 and a stroke volume determined by using the signal measured by the measurement device 8 and the model to be trained is reduced, particularly minimized.

The system 1 for determining the physiological parameter of the subject can be configured to remotely monitor the heart function. For instance, heart failure can be monitored directly. Heart failure is a defect in the pumping function of the heart, for instance, the heart is not able to pump sufficient blood into the surrounding tissue which can lead to symptoms such as lung edema, sudden weight increase, tiredness and ultimate damage to the heart and other tissues. After a first treatment in a hospital, heart failure patients are very often re-hospitalized when symptoms of heart failure reoccur. Over 50 % of all heart failure patients are re-hospitalized after six months of initial treatment. Heart failure is the leading cause of hospitalization in adults over 65 years in the U.S. Reoccurrence of heart failure is noticed when patients show symptoms, which is already too late, by then the function of the heart has deteriorated further. By using the above described system 1 for determining a physiological parameter of a subject, it is possible to measure heart failure before symptoms occur, wherein the patient's medication or lifestyle then can be adapted to prevent re-hospitalization. Since the above described system for determining a physiological parameter of a subject is sensitive to tissue deformation and changes in blood volume, the system can be used to sense changes in the heart pumping function, unlike, for instance, ECG which is not directly sensitive to this but only measures heart rhythm, but not the heart pumping function.

The system also can be configured to monitor cardiac failure indirectly through detection of lung edema. Cardiac failure patients often suffer from lung edema as a result of cardiac failure. If the patients show symptoms of lung edema, there is already significant damage done to the heart and lungs. The system can be configured such that the provided motion signal is related to the mechanical movement of the lungs within the subject, wherein in this case the signal is very sensitive to respiratory motion. Since with developing lung edema the motion of the lungs changes, by monitoring the movement of the lungs, developing lung edema can be detected, thereby indirectly detecting cardiac failure. In this example, the determined physiological parameter can be a characteristic of the movement of the lungs like the frequency or amplitude of this movement.

If the system is configured to provide a motion signal that is related to a mechanical movement of the heart within a subject and to use this signal to determine a heart-related physiological parameter like the stroke volume or the heart rate, the heart-related physiological parameter can be used to monitor arrhythmia in cardiovascular patients. Such monitoring is normally done by using ECG measurements. However, ECG measurements use electrodes that are attached to the skin which is uncomfortable for patients. The above described system for determining a heart-related physiological parameter of the subject does not need to be attached to the skin, thus improving patient comfort.

The system can also be configured to remotely monitor lung ventilation. In particular, the measurement device can be configured to provide a motion signal that is related to the mechanical movement of the lungs within a subject, wherein the model can be trained such that, given the motion signal, a lung-related physiological parameter measured by, for instance, spirometry or MRI is output. The processor of the determination device then can determine a lung-related physiological parameter based on the provided motion signal and the trained model. In this case, for training the model a spirometry system or MRI system can be used.

The system can also be used for tracking catheters during implantation. During heart catheterization, generally a long thin tube is inserted in an artery or vein and threaded to the heart where it is used to treat or diagnose certain heart diseases. These catheters contain electrically conductive materials which makes RF measurements very sensitive to the position and movement of these wires. The resulting motion signal, which is related to the mechanical movement of the catheters, can be used for determining a physiological parameter like the stroke volume.

The system can also be configured to measure a heart-related physiological parameter like the heart rate or a lung-related physiological parameter like the breathing rate during physical exercise. It is known to do this with ECG which needs to make contact with the skin by using electrodes. In contrast to this, the above described system can measure the heart-related or lung-related physiological parameters without needing to make contact with the skin.

Although in above described embodiments the model mainly is a linear model, the model can also be another one. Generally, the model can be any relation between a) a physiological parameter like the stroke volume or a ventilation parameter and b) the motion signal provided by the measurement device. Such a relation could be determined by calibration/training, but also by electromagnetic simulation. For instance, for different distributions and dimensions of human components like organs, bones, skin, et cetera a respective electromagnetic simulation can be carried out and hence a respective relation, i.e. model, can be determined. Based on a specific distribution and specific dimensions of, for instance, the organs, the bones, the skin, et cetera of a respective subject, which might be known based on an image of the respective subject like an MRI, CT, ultrasound et cetera image, a matching model can be selected and used for determining the physiological parameter based on the motion signal. For carrying out the electromagnetic simulation, finite difference time domain simulations can be used. This can be done with commercially available electromagnetic solvers such as shown in the article by Navest et al., Magnetic Resonance in Medicine, 2019, 82:6 (2236-2247) which is herewith incorporated by reference.

In an embodiment, the relations and hence the models, which have been determined by electromagnetic simulation, together with body parameters describing the respective distributions and dimensions of human components like organs, bones, skin, et cetera can be used to train an artificial intelligence (Al). The body parameters could be, for instance, a dimension of the torso like its circumference and the Al can be trained such that, given one or several body parameters and the motion signal provided by the measurement device, the physiological parameter is output. Different Al methods could be used, for example regression models, Gaussian processes, neural networks, k-nearest neighbors or support vector machine. In an embodiment, scalar parameters such as body circumference, stroke volume at rest, BMI, age or sex are specified as input to the model. Moreover, in an embodiment, a model of the dielectric property distribution in the area of interest like the torso of the subject is obtained based on MRI, CT or ultrasound imaging. The dielectric property distribution can be provided as an input to train the Al and later to update the model.

In a further embodiment a specific distribution and specific dimensions of human components like organs, bones, skin, et cetera of the subject, of whom the relation between the motion signal provided by the measurement device and the physiological parameter should be determined, are determined based on an image of the subject like a CT or MR image, wherein the relation, i.e. the model, can be determined based on an electromagnetic simulation applied to the determined specific distribution and specific dimensions of the human components.

Hence, a system for determining a physiological parameter like a stroke volume of the heart of a subject is provided. A measurement device includes a) an RF antenna module and b) an RF instrument configured to transmit RF power into the RF antenna module, to receive an RF signal from the RF antenna module and to provide a motion signal that is related to a mechanical movement of a structure within the subject based on the received RF signal. A first RF antenna and a second RF antenna of the RF antenna module are used in an opposing configuration such that electromagnetic phase profiles of the RF antennas at least partly cancel out, wherein the subject is located in between the opposing RF antennas. The physiological parameter is determined based on the provided motion signal.

Although in some embodiments described above the measurement device is configured to be worn by the subject, it is also possible that the measurement device is not configured to be worn by the subject. For instance, the measurement device can also be configured to be arranged on a wall or to be arranged on a rack, stage or the like, wherein the subject can be arranged in between the opposing RF antennas of the measurement device for determining the physiological parameter.

Although in above described embodiments, the first and second RF antennas have a certain construction, the first and second RF antennas can also be constructed in another way. For instance, the first and/or second RF antenna can be a loop antenna. Furthermore, at least one of the RF antennas can be a dipole antenna like the so-called "coax dipole antenna" disclosed in the article "The Coax Dipole: A fully flexible coaxial cable dipole antenna with flattened current distribution for body imaging at 7 Tesla" by C. C. van Leeuwen et al., Magnetic Resonance in Medicine, Volume 87, Issue 1, pages 528-540 (2022), which is herewith incorporated by reference.

Although in above described embodiments, the terms "motion signal" or "processed motion signal" refer to specific signals corresponding to the embodiments, a motion signal in general can be a signal obtained by receiving and processing an RF signal that carries information about the mechanical motion of the structure within the object. While the receiving of the RF signal is performed by the measuring device, the processing of the RF signal can be performed by several elements or units, in particular the RF instrument and/or the processor of the determination device.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the determination of the physiological parameter, the training of the model, et cetera performed by one or several units or devices can be performed by any other number of units or devices. These procedures and/or the control of the components of the system for determining the physiological parameter of the subject in accordance with the above described method for determining the physiological parameter of the subject and/or the control of the training system in accordance with the training method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. A system for determining a physiological parameter of a subject (7), the system (1) comprising:
- a measurement device (8) including a) an RF antenna module (3) comprising a first RF antenna (4) and a second RF antenna (5) and b) an RF instrument (2) connected to the RF antenna module (3) and configured to transmit RF power into the RF antenna module (3), to receive an RF signal from the RF antenna module (3) and to provide a motion signal that is related to a mechanical movement of a structure (6) within the subject (7) based on the received RF signal,
- a determination device (12) configured to determine the physiological parameter based on the provided motion signal, wherein the determination device (12) comprises a model providing module (14) configured to provide a model that provides, as an output, a physiological parameter if, as an input, a motion signal is provided, and a processor (15) configured to determine the physiological parameter based on the provided model and the provided motion signal,
wherein the RF antenna module (3) is configured to use the RF antennas (4, 5) in an opposing configuration such that electromagnetic phase profiles of the RF antennas (4, 5) at least partly cancel out each other, wherein in use the subject (7) is located in between the opposing RF antennas (4, 5).

2. The system as defined by claim 1, wherein the RF antennas (4, 5) are dipole antennas.

3. The system as defined by claim 2, wherein the dipole antennas (4, 5) are folded.

4. The system as defined by claim 3, wherein at least one end of the respective dipole antenna (4, 5) is folded.

5. The system as defined by claim 4, wherein the length of the folded end is within a range from 40% to 49.5% of the overall length of the respective dipole antenna (4, 5).

6. The system as defined by any of claims 4 and 5, wherein the at least one end of the respective dipole antenna (4, 5) is folded such that in use, when the subject is located in between the opposing RF antennas (4, 5), it is folded away from the subject.

7. The system as defined by any of claims 4 to 6, wherein the RF antennas are folded on a dielectric substrate.

8. The system as defined by any of the preceding claims, wherein the RF antenna module (3) is configured such that the operating frequency of the RF antennas (4, 5) is lower than 1 GHz and further preferred lower than 600 MHz.

9. The system as defined by any of the preceding claims, wherein the RF antenna module (3) is configured to use the first RF antenna (4) for transmission and the second RF antenna (5) for reception or vice versa.

10. The system as defined by any of the preceding claims, wherein the RF antenna module (3) is configured such that the electromagnetic phase profiles of the opposing RF antennas (4, 5) are symmetric in the sense that negative phases of an electromagnetic phase profile of the first RF antenna (4) overlap with positive phases of an electromagnetic phase profile of the second RF antenna (5) and vice versa.

11. The system as defined by any of the preceding claims, wherein at least one of the RF antennas (4, 5) comprises a capacitor and/or an inductor like a coil, which are connected in series by using conductors.

12. The system as defined by claim 11, wherein the capacitor has a capacity in a range from 2 to 10 pF and/or the inductor has an inductivity in a range from 5 to 15 nH.

13. The system as defined by any of claims 11 and 12, wherein the respective RF antenna comprises several capacitors and several inductors.

14. A measurement device including a) an RF antenna module (3) comprising a first RF antenna (4) and a second RF antenna (5) and b) an RF instrument (2) connected to the RF antenna module (3) and configured to transmit RF power into the RF antenna module (3) and to receive an RF signal from the RF antenna module (3), wherein the RF antenna module (3) is configured to use the RF antennas (4, 5) in an opposing configuration such that electromagnetic phase profiles of the RF antennas (4, 5) at least partly cancel out each other, wherein in use a subject is located in between the opposing RF antennas.

15. A set of a first RF antenna (4) and a second RF antenna (5), wherein the RF antennas (4, 5) are configured to be used in an opposing configuration such that electromagnetic phase profiles of the RF antennas (4, 5) at least partly cancel out each other, wherein in use a subject is located in between the opposing RF antennas.
